# EUROPEAN PATENT APPLICATION

(11) **EP 4 053 115 A1**
(43) Date of publication of application: **07.09.2022**
(21) Application number: 21305244.2
(22) Date of filing: 01.03.2021
(51) Int. Cl.: C07D 335/16, C07C 69/00, C08F 2/00, G03F 7/00

(54) **LIQUID TYPE-II PHOTOINITIATORS**

(71) Applicant: ARKEMA FRANCE, 92700 Colombes (FR)
(72) Inventor: JEPSON, David, Wetherby, West Yorkshire LS22 7NS (GB); SQUIRES, Kelly, Wetherby, West Yorkshire LS22 7NS (GB); PLENDERLEITH, Richard, Wetherby, West Yorkshire LS22 7NS (GB)
(74) Representative: Arkema Patent

(57) **Abstract**

The present invention relates to novel Norrish Type II photoinitiators which may be liquid or easily solubilized at room temperature. The invention also relates to compositions comprising these photoinitiators as well as their use for photopolymerizing ethylenically unsaturated compounds or solubilizing other photoinitiators.

## Description

### Field of the Invention

The present invention relates to novel Norrish Type II photoinitiators which may be liquid or easily solubilized at room temperature. The invention also relates to compositions comprising these photoinitiators as well as their use for photopolymerizing ethylenically unsaturated compounds or solubilizing other photoinitiators.

### Background of the Invention

Radiation curable compositions containing ethylenically unsaturated compounds can be polymerised by exposure to radiation, such as ultraviolet (UV) light. For rapid and effective curing, a photoinitiator is often used. The photoinitiator forms radical species upon irradiation with photons and initiates free-radical polymerisation of unsaturated groups, leading to hardening (curing) of the material.

Free radical photoinitiators can adopt two different modes of action, and are classified by mode of action as Norrish Type I and Norrish Type II Photoinitiators. Norrish Type I photoinitiators cleave upon exposure to radiation, producing radical species which are capable of initiating the polymerisation of unsaturated compounds. Norrish Type II photoinitiators are compounds which do not fragment upon exposure to radiation and so will not typically initiate radical-chain polymerisation unless a co-initiator is present. Upon exposure to radiation, interaction between the Type II photoinitiator and the co-initiator leads to the generation of radical species which can initiate the polymerisation of UV-curable resins.

The most common Type-II photoinitiators are diaryl ketones such as benzophenones (e.g. SpeedCure^{®} BP available from Arkema) or thioxanthones such as 2-isopropylthioxanthone (SpeedCure^{®} 2-ITX available from Arkema) or diethylthioxanthone (SpeedCure^{®} DETX available from Arkema).

However, most of the commercially available Type-II photoinitiators are solids at room temperature (20-25°C). The introduction of solid photoinitiators in UV-curable formulations may lead to solubility and viscosity issues which can limit their use in specific applications such as inkjet printing. Indeed, inkjet ink formulations should exhibit low viscosity to allow rapid printing speed and they should not contain any particulates to avoid clogging of the nozzles.

It would therefore desirable to provide novel photoinitiators which may advantageously be liquid or easily solubilized in inkjet formulation, in particular liquid benzophenones and thioxanthones, which can be used in the photopolymerization of ethylenically unsaturated compounds, whilst maintaining or improving curing performance relative to known systems.

Novel thioxanthones would be of particular interest since they are curable by UV radiation from light emitting diode (LED) light sources due to their emission band in the range of 365-420 nm. UV-LED curing is advantageous since LEDs are more compact, less expensive and more environmentally-friendly than broad spectra mercury lamps. However, the use of LEDs is challenging due to increased oxygen inhibition which may limit surface curing and hence the performances of the resulting cured product. The novel thioxanthone photoinitiators should therefore advantageously exhibit good photochemical activity, and low oxygen sensitivity.

The invention is intended to overcome or ameliorate at least some aspects of this problem.

### Summary of the Invention

A first aspect of the invention is a compound of general formula (I) 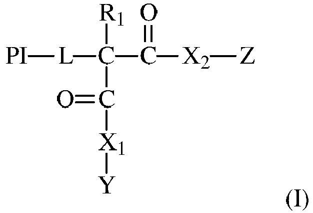 wherein PI, L, R₁, X₁, X₂, Y and Z are as defined herein.

Another aspect of the invention is a photoinitiator composition comprising a mixture of at least two distinct compounds of formula (I) according to the invention.

Another aspect of the invention is a photoinitiator composition comprising a compound of formula (I) according to the invention and a photoinitiator other than a compound of formula (I).

The invention also aims to provide a process for photopolymerizing one or more ethylenically unsaturated compounds, the process comprising contacting one or more ethylenically unsaturated compounds with a compound of formula (I) according to the invention or a photoinitiator composition according to the invention, and irradiating the mixture, in particular with visible, near-UV and/or UV light, more particularly with a LED light source.

Another aspect of the invention is a curable composition comprising
a) a compound of formula (I) according to the invention or a photoinitiator composition according to the invention; and
b) an ethylenically unsaturated compound.

Another aspect of the invention is a process for the preparation of a cured product, comprising curing the curable composition according to the invention, in particular by exposing the curable composition to radiation such as UV, near-UV and/or visible radiation, more particularly by exposing the curable composition to a LED light source.

Another aspect of the invention is a process of inkjet printing comprising jetting the curable composition of the invention onto a substrate.

Another aspect of the invention is a use of a compound of formula (I) according to the invention or a photoinitiator composition according to the invention, as a photoinitiator or a photoinitiating system, in particular as a photoinitiator or a photoinitiating system in a radiation curable composition, in particular in a UV or LED-curable composition.

Another aspect of the invention is a use of a compound of formula (I) according to the invention or a photoinitiator composition according to the invention, in a photopolymerization reaction.

Another aspect of the invention is a use of a compound of formula (I) according to the invention or a photoinitiator composition according to the invention, to solubilize a photoinitiator other than a compound of formula (I).

### Detailed Description

### Definitions

In the present application, the term "comprise(s) a/an" means "comprise(s) one or more".

Unless mentioned otherwise, the % by weight in a compound or a composition are expressed based on the weight of the compound, respectively of the composition.

The term "photoinitiator moiety" means a moiety capable of generating radical species upon irradiation with photons, optionally in the presence of a co-initiator such as a tertiary amine.

The term "arylketone moiety" means a moiety comprising an aryl substituted with a -C(=O)-group. In particular, an arylketone moiety may be a moiety comprising two aryls linked together by a -C(=O)- group, the aryls being optionally further linked together by another group.

The term « aryl » means an optionally substituted polyunsaturated aromatic group. The aryl may contain a single ring (i.e. phenyl) or more than one ring wherein at least one ring is aromatic. When the aryl comprises more than one more ring, the rings may be fused, linked via a covalent bond (for example biphenyl). The aromatic ring may optionally comprise one to two additional fused rings (i.e. cycloalkyl, heterocycloalkyl or heteroaryl). The term "aryl" also encompasses partially hydrogenated derivatives of the carbocyclic system is described above. Examples include phenyl, naphtyl, biphenyl, phenanthrenyl and naphthacenyl.

The term « alkyl » means a monovalent saturated alicyclic hydrocarbon group of formula -CₙH₂ₙ₊₁ (wherein n is from 1 to 200). An alkyl may be linear or branched. A « C1-C6 alkyl » means an alkyl having 1 to 6 carbon atoms. Examples of alkyl groups include methyl, ethyl, propyl, isopropyl, butyl, isobutyl, tert-butyl, pentyl, hexyl, 2-methylbutyl, 2,2-dimethylpropyl, n-hexyl, 2-methylpentyl, 2,2-dimethylbutyl, n-heptyl, 2-ethylhexyl, and the like.

The term « halogen » means an atom selected from C1, Br, F and I.

The term « cycloalkyl » means a monovalent saturated alicyclic hydrocarbon group comprising a cycle. Examples of cycloalkyl groups include cyclopentyl, cyclohexyl and isobornyl.

The term « heterocycloalkyl » means a cycloalkyl having at least one ring atom that is a heteroatom selected from O, N or S.

The term « alkoxy » means a group of formula -O-alkyl, wherein the alkyl is as defined above.

The term « aryloxy » means a group of formula -O-aryl, wherein the aryl is as defined above.

The term « thioalkyl » means a group of formula -S-alkyl, wherein the alkyl is as defined above.

The term « thioaryl » means a group of formula -S-aryl, wherein the aryl is as defined above.

The term « alkenyl » means a monovalent alicyclic hydrocarbon group comprising at least one C=C double bond. An alkenyl may be linear or branched.

The term « alkynyl » means a monovalent alicyclic hydrocarbon group comprising at least one C≡C triple bond. An alkynyl may be linear or branched.

The term « aralkyl » means an aryl substituted by an alkyl group. An example of an aralkyl group is tolyl.

The term « alkaryl » means an alkyl substituted by an aryl group. An example of an alkaryl group is benzyl (-CH2-Phenyl).

The term « heteroaryl » means an aryl having at least one ring atom that is a heteroatom selected from O, N or S.

The term « alkylamino » means an alkyl substituted by at least one amino group.

The term « alkylthiol » means an alkyl substituted by at least one thiol group.

The term « hydroxyalkyl » means an alkyl substituted by at least one hydroxy group.

The term « haloalkyl » means an alkyl substituted by at least one halogen.

The term « alkylene » or « alkanediyl » means a linker derived from an alkane of formula CₘH₂ₘ₊₂ (wherein m is 1 to 200) by removing one hydrogen atom at each point of attachment of the linker. A « C2-C20 alkylene » means an alkylene having 2 to 20 carbon atoms.

The term « thioalkylene » means an alkylene interrupted by one or more thioether bonds.

The term « ketoalkylene » means an alkylene substituted by one or more oxo (=O) groups.

The term « alkenylene » means an alkylene linker comprising one or more C=C double bonds.

The term « arylene » means a linker derived from an aryl by removing one hydrogen for each point of attachment of the linker.

The term « oxyalkylene » means a linker comprising one or more -R-O- or -O-R- units, wherein R is an alkylene. Examples of oxyalkylenes include oxyethylene (-O-CH₂-CH₂-), oxypropylene (-O-CH₂-CH(CH₃)- or -O-CH(CH₃)-CH₂-) and oxybutylene (-O-CH2-CH2-CH2-CH2-), polyoxyethylene (-[O-CH₂-CH₂]ₙ-), polyoxypropylene (-[O-CH₂-CH(CH₃)]ₙ- or -[O-CH(CH₃)-CH₂]ₙ-) and polyoxybutylene (-[O-CH₂-CH₂-CH₂-CH₂]ₙ-) where n is 2 to 100.

The term "alkoxylated" means a compound, group or linker containing one or more oxyalkylene moieties, in particular one or more oxyethylene and/or oxypropylene moieties. For example, an alkoxylated compound, group or linker may contain from 1 to 30 oxyalkylene moieties.

The term « linker » means a plurivalent group. A linker may connect at least two moieties of a compound together, in particular 2 to 16 moieties of a compound together. For example, a linker that connects two moieties of a compound together is referred to as a divalent linker, a linker that connects three moieties of a compound together is referred to as a trivalent linker.

The term « hydrocarbon linker » means a linker having a carbon backbone chain which may optionally be interrupted by one or more heteroatoms selected from N, O, S, Si and mixtures thereof. A hydrocarbon linker may be aliphatic, cycloaliphatic or aromatic. A hydrocarbon linker may be saturated or unsaturated. A hydrocarbon linker may be optionally substituted.

The term « aliphatic » means a non-aromatic acyclic compound. It may be linear or branched, saturated or unsaturated. It may be substituted by one or more groups, for example selected from alkyl, hydroxyl, halogen (Br, Cl, I), isocyanate, carbonyl, amine, carboxylic acid, -C(=O)-OR', -C(=O)-O-C(=O)-R', each R' being independently a C1-C6 alkyl. It may comprise one or more bonds selected from ether, ester, amide, urethane, urea and mixtures thereof.

The term « acyclic » means a compound that does not comprise any rings

The term « cycloaliphatic » means a non-aromatic cyclic compound. It may be substituted by one or more groups as defined for the term « aliphatic ». It may comprise one or more bonds as defined for the term « aliphatic ».

The term « aromatic » means a compound comprising an aromatic ring, which means that respects Hückel's aromaticity rule, in particular a compound comprising a phenyl group. It may be substituted by one or more groups as defined for the term « aliphatic ». It may comprise one or more bonds as defined for the term « aliphatic ».

The term « saturated » means a compound that does not comprise any double or triple carbon-carbon bonds.

The term « unsaturated » means a compound that comprises a double or triple carbon-carbon bond, in particular a double carbon-carbon bond.

The term « polyol » means a compound comprising at least two hydroxyl groups.

The term « polyether polyol » or « polyether linker » means a polyol, respectively a linker, comprising at least two ether bonds.

The term « polyester polyol » or « polyester linker » means a polyol, respectively a linker, comprising at least two ester bonds.

The term « polycarbonate polyol » or « polycarbonate linker » means a polyol, respectively a linker, comprising at least two carbonate bonds.

The term « polyurethane linker » means a linker comprising at least two urethane bonds.

The term « polysulfide linker » means a linker comprising at least two thioether bonds.

The term « polyorganosiloxane polyol » or « polyorganosiloxane linker » means a polyol, respectively a linker, comprising at least two organosiloxane bonds. The organosiloxane may, for example be a dimethylsiloxane bond.

The term « polyamine linker » means a linker comprising at least two amine bonds.

The term « polycaprolactone polyol » or « polycaprolactone linker » means a polyol, respectively a linker, comprising at least two units derived from the ring-opening polymerization of ε-caprolactone, in particular at least two -[(CH₂)₅-C(=O)O]- units.

The term « polybutadiene polyol » or « polybutadiene linker » means a polyol, respectively a linker, comprising at least two units derived from the polymerization of butadiene, in particular at least two units selected from -CH₂-CH=CH-CH₂- and CH₂-CH(CH=CH₂)-.

The term « isocyanurate linker » means a linker comprising an isocyanurate moiety, in particular a moiety of formula:

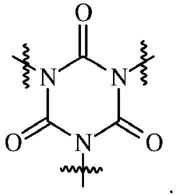

The term « hydroxyl group » means a -OH group.

The term « thiol group » means a -SH group.

The term « amino group » means a -NRₐ₁R_{b1} group, wherein Rₐ₁ and R_{b1} are independently H or an optionally substituted alkyl. A « primary amino group » means a -NRₐ₁R_{b1} group, wherein Rₐ₁ and R_{b1} are H. A « secondary amino group » means a -NRₐ₁R_{b1} group, wherein Rₐ₁ is H and R_{b1} is an optionally substituted alkyl.

The term « carboxylic acid » means a-COOH group.

The term « isocyanate group » means a -N=C=O group.

The term « ester bond » means a -C(=O)-O- or -O-C(=O)- bond.

The term « ether bond » means a -O- bond.

The term « thioether bond » means a -S- bond.

The term « organosiloxane bond » means a -Si(R_{c1})₂-O- bond, wherein R_{c1} is an organic group, in particular an organic group selected from alkyl, alkoxy and aryl. The term « dimethylsiloxane bond » means a -Si(CH₃)2-O- bond

The term « carbonate bond » means a -O-C(=O)-O- bond.

The term « urethane or carbamate bond » means a -NH-C(=O)-O- or -O-C(=O)-NH- bond.

The term « amide bond » means a -C(=O)-NH- or -NH-C(=O)- bond.

The term « urea bond » means a -NH-C(=O)-NH- bond.

The term « polyisocyanate » means a compound comprising at least two isocyanate groups.

The term « amine bond » means a -N(R_{d1})- bond, wherein R_{d1} is independently H or a C1-C6 alkyl optionally substituted by an amino group.

The term « optionally substituted » means a compound, group or linker optionally substituted by one or more groups selected from halogen, alkyl, cycloalkyl, aryl, heteroaryl, alkoxy, aryloxy, aralkyl, alkaryl, haloalkyl, hydroxyl, thiol, hydroxyalkyl, thioalkyl, thioaryl, alkylthiol, amino, alkylamino, isocyanate, nitrile, amide, carboxylic acid, -C(=O)-R' -C(=O)-OR', -C(=O)NH-R', -NH-C(=O)R', -O-C(=O)-NH-R', -NH-C(=O)-O-R', -C(=O)-O-C(=O)-R' and -SO₂-NH-R', each R' being independently an optionally substituted group selected from alkyl, aryl and alkylaryl.

### Compound of formula (I)

The compound of the invention corresponds to the following formula (I): 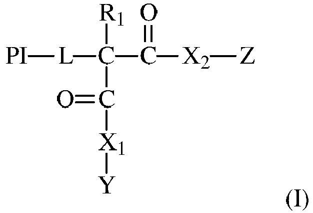 wherein
each PI is independently a photoinitiator moiety comprising an arylketone moiety;
each L is independently a linker selected from a direct bond, -C(=O)- or a divalent linker comprising 1 to 6, in particular 1 to 4, more particularly 1 or 2, carbon atoms;
each R₁ is independently H, alkyl or aryl;
each X₁ and X₂ is independently O, NR₂, N(R₂)₃, or S;
each R₂ is independently H or an optionally substituted alkyl;
each Y and Z are independently selected from H, an ionic moiety and an organic group, or Y and Z may form, together with the atoms to which they are attached, a ring.

The compound of formula (I) bears at least one photoinitiator moiety PI. In one embodiment, the compound of formula (I) bears a single photoinitiator moiety PI. In another embodiment, the compound of formula (I) bears at least two photoinitiator moieties PI, which may be identical or different.

Each PI is independently a photoinitiator moiety comprising an arylketone moiety. The arylketone moiety may be selected from a thioxanthone moiety, a benzophenone moiety, an anthraquinone moiety, a xanthone moiety, an anthrone moiety and an acridone moiety.

Each PI may independently be a photoinitiator moiety of formula (II) or (III): 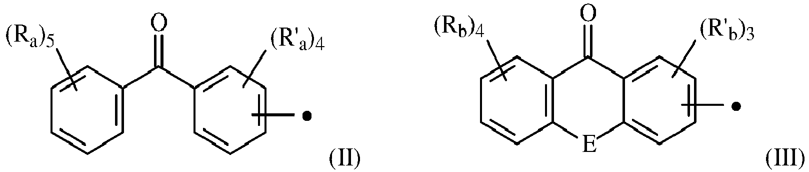 wherein
E is S, O, C(=O), CRoR'o or NR₃, in particular S;
R₀, R'₀ and R₃ are independently H, an optionally substituted alkyl or an optionally substituted aryl;
the symbol • represents a point of attachment to linker L of formula (I);
Rₐ, R'ₐ, R_{b} and R'_{b} are independently selected from H, halogen, alkyl, cycloalkyl, heterocycloalkyl, alkoxy, aryloxy, thioalkyl, thioaryl, alkenyl, alkynyl, aryl, aralkyl, alkaryl, heteroaryl, -C(=O)R₄, -NR₅R₆, alkylamino, alkylthiol, hydroxyalkyl, haloalkyl, -NO₂, -CN, -C(=O)OR₇, -C(=O)NR₅R₆, -OH, -SH, and a group of formula (IV): 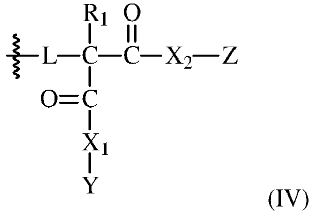
wherein L, R₁, X₁, X₂ ,Y and Z are as defined herein;
two adjacent Rₐ, R'ₐ, R_{b} and R'_{b} groups may form, with the carbon atoms to which they are attached, a 5 to 8 membered ring;
R₄ is selected from an optionally substituted alkyl, an optionally substituted cycloalkyl, an optionally substituted heterocycloalkyl and an optionally substituted aryl;
R₅, R₆ and R₇ are independently selected from H, alkyl and aryl.

In particular, each PI may independently be a photoinitiator moiety of formula (II) or (III) as defined above, wherein
E is S;
Rₐ, R'ₐ, R_{b} and R'_{b} are independently selected from H, halogen, alkyl, alkoxy, thioalkyl, aryl, -C(=O)R₄, -NR₅R₆, -OH and -SH, and two adjacent Rₐ, R'ₐ, R_{b} and R'_{b} groups may form, with the carbon atoms to which they are attached, a 5 to 8 membered ring.

Even more particularly, each PI may independently be a photoinitiator moiety of formula (II) or (III) as defined above, wherein
E is S;
Rₐ, R'ₐ, R_{b} and R'_{b} are all H.

In one embodiment, each PI is independently a photoinitiator moiety comprising an arylketone moiety selected from a thioxanthone moiety and a benzophenone moiety. Each PI may independently be a benzophenone moiety of formula (IIa), (IIb), (IIc) or (IId) or a thioxanthone moiety of formula (IIIa), (IIIb), (IIIc) or (IIId): 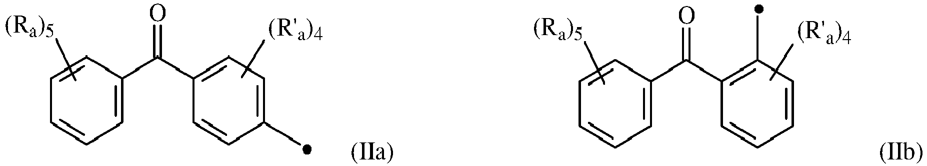 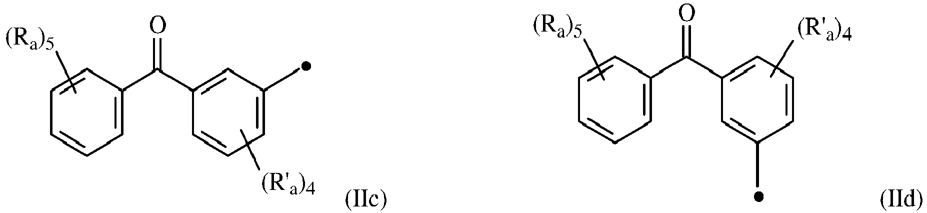 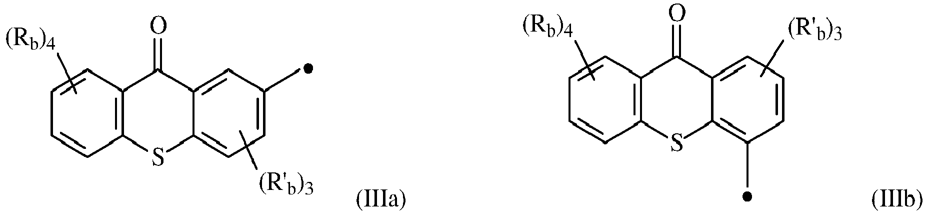 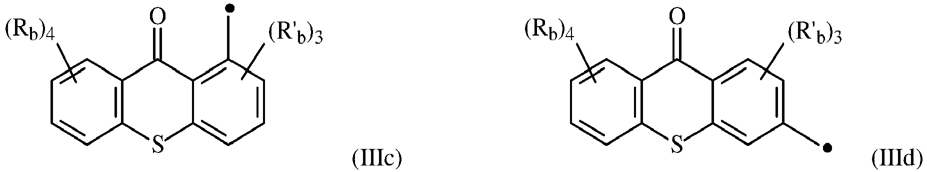 wherein Rₐ, R'ₐ, R_{b}, R'_{b} and symbol • are as defined above.

In particular, each PI may independently be a benzophenone moiety of formula (IIa), (IIb), (IIc) or (IId) or a thioxanthone moiety of formula (IIIa), (IIIb), (IIIc) or (IIId) as defined above wherein
Rₐ, R'ₐ, R_{b} and R'_{b} are independently selected from H, halogen, alkyl, alkoxy, thioalkyl, aryl, -C(=O)R₄, -NR₅R₆, -OH and -SH, and two adjacent Rₐ, R'ₐ, R_{b} and R'_{b} groups may form, with the carbon atoms to which they are attached, a 5 to 8 membered ring.

More particularly, each PI may independently be a benzophenone moiety of formula (IIa), (IIb), (IIc) or (IId) or a thioxanthone moiety of formula (IIIa), (IIIb), (IIIc) or (IIId) as defined above wherein Rₐ, R'ₐ, R_{b} and R'_{b} are all H.

In one embodiment, each PI may independently be a benzophenone moiety of formula (IIa) or (IIb) or a thioxanthone moiety of formula (IIIa) or (IIIb) as defined above.

In particular, each PI may independently be a benzophenone moiety of formula (IIa) or (IIb) or a thioxanthone moiety of formula (IIIa) or (IIIb) as defined above wherein Rₐ, R'ₐ, R_{b} and R'_{b} are independently selected from H, halogen, alkyl, alkoxy, thioalkyl, aryl, -C(=O)R₄, -NR₅R₆, -OH and -SH, and two adjacent Rₐ, R'ₐ, R_{b} and R'_{b} groups may form, with the carbon atoms to which they are attached, a 5 to 8 membered ring.

More particularly, each PI may independently be a benzophenone moiety of formula (IIa) or (IIb) or a thioxanthone moiety of formula (IIIa) or (IIIb) wherein Rₐ, R'ₐ, R_{b} and R'_{b} are all H.

Each PI is connected to a linker L. Each L is independently a linker selected from a direct bond, -C(=O)- or a divalent linker comprising 1 to 6, in particular 1 to 4, more particularly 1 or 2, carbon atoms.

In particular, each L may independently be a linker selected from direct bond, -(C=O)-, alkylene, oxyalkylene, thioalkylene, ketoalkylene, alkenylene, arylene.

More particularly, each L may independently be a linker selected from a direct bond, -(C=O)-, an alkylene of formula (V), an oxyalkylene of formula (VI), a thioalkylene of formula (VII) and a ketoalkylene of formula (VIII) 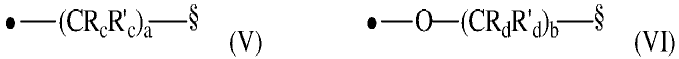 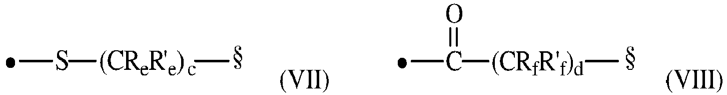 wherein
each R_{c}, R'_{c}, R_{d}, R'_{d}, Rₑ, R'ₑ, R_{f} and R'_{f} is independently H or alkyl, in particular H;
a, b and c are independently 1, 2, 3, 4, 5 or 6, in particular 1 or 2, more particularly 1;
d is 1, 2, 3, 4 or 5, in particular 1 or 2, more particularly 1
the symbol • represents a point of attachment to the PI moiety of formula (I);
the symbol § represents a point of attachment to the C(R₁) moiety of formula (I).

Even more particularly, each L may independently be an alkylene of formula (V) wherein
R_{c} and R'_{c} are independently H or alkyl, in particular H;
a is 1, 2, 3, 4, 5 or 6, in particular 1 or 2, more particularly 1.

Each linker L is connected to a C(R₁) moiety. The R₁ group of each C(R₁) moiety is independently H, alkyl or aryl. In particular, R₁ is H.

Each C(R₁) moiety is linked to both a C(=O)-X₁ moiety and a C(=O)-X₂ moiety. Each X₁ and X₂ is independently O, NR₂ or S and each R₂ is independently H or an optionally substituted alkyl. Accordingly, X₁ and X₂ may be selected so as to form, with the -C(=O)- group to which they are attached, an ester bond, an amide bond or a thioester bond.

In one embodiment, X₁ and X₂ are both NR₂. In another embodiment, X₁ and X₂ are both S. In a preferred embodiment, X₁ and X₂ are both O.

Each C(=O)-X₁ moiety is linked to a Z moiety and each C(=O)-X₂ moiety is linked to a Y moiety.

Each Y and Z is independently selected from H, an ionic moiety and an organic group, or Y and Z may form, together with the atoms to which they are attached, a ring.

Y and Z may be identical or different. In one embodiment, Y and Z are identical. In another embodiment, Y and Z are different.

Each Y and Z may independently be selected from H, an ionic moiety, an optionally substituted alkyl, an alkoxylated alkyl, a polymeric backbone, a polymeric core and a group of formula (IX): 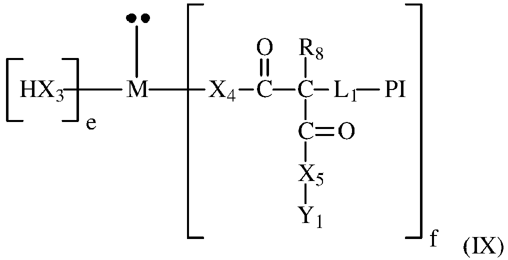 wherein
M is a (e+f)valent linker;
each PI is independently a photoinitiator moiety comprising an arylketone;
each L₁ is independently a linker selected from a direct bond, -C(=O)- or a divalent linker comprising 1 to 6, in particular 1 to 4, more particularly 1 or 2, carbon atoms;
each R₈ is independently H, alkyl or aryl;
each X₃, X₄ and X₅ is independently O, NR₉ or S;
each R₉ is independently H or an optionally substituted alkyl;
each Y₁ is independently H, an optionally substituted alkyl, an alkoxylated alkyl or Y₁ may form a ring with Y, Z or another Y₁;
e is from 0 to 6;
f is from 0 to 6;
with the proviso that at least one of e and f is not 0;
symbol •• represents a point of attachment to X₁ or X₂.

Part or all of the Y moieties and/or part of all of the Z moieties may be a hydrogen atom. In one embodiment, all of the Y moieties and all of the Z moieties are a hydrogen atom. In another embodiment, all of the Y moieties are a hydrogen atom and none of the Z moieties are a hydrogen atom.

Part or all of the Y moieties and/or part of all of the Z moieties may independently be an ionic moiety. In one embodiment, all of the Y moieties and all of the Z moieties are independently an ionic moiety. In another embodiment, all of the Y moieties are independently an ionic moiety and none of the Z moieties are an ionic moiety.

When X₁, respectively X₂, is O or S, then Y, respectively Z, may be a cationic moiety. When Y, respectively Z, is a cationic moiety, then X₁, respectively X₂, bears a negative charge and the bond between X₁ and Y, respectively the bond between X₂ and Z, is an ionic bond. The cationic moiety may, in particular, comprise a metal such as an alkali metal, an alkaline earth metal, a transition metal, a metalloid or a semimetal. Examples of suitable metals include Li, Na, K, Ce, Mg, Ca, Sr, Ba, Ti, Zr, V, Cr, Mo, W, Mn, Fe, Co, Ni, Pd, Cu, Zn, Cd, Hg, B, Al, Ga, In, Si, Ge, Sn, Pb, Sb and Bi. If the metal is not monovalent, the cationic moiety may further comprise one or more anionic moieties and optionally one or more metals so that the compound of formula (I) is electrically neutral. For example, if Z is divalent metal M, the compound of formula may correspond to the following formula:

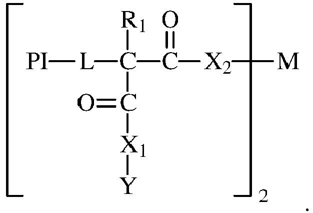

When X₁, respectively X₂, is N(R₂)₃ then Y, respectively Z, is an anionic moiety. When Y, respectively Z, is an anionic moiety, then X₁, respectively X₂, bears a positive charge and the bond between X₁ and Y, respectively the bond between X₂ and Z, is an ionic bond. The anionic moiety may, in particular, be a halide (such as chloride, bromide, iodide, fluoride), a phosphate, a sulfate, a phosphonate, a sulfonate, a hydroxide, an alkoxide or a carboxylate.

Part or all of the Y moieties and/or part of all of the Z moieties may independently be an optionally substituted alkyl, hereinafter referred to as Alk. In one embodiment, all of the Y moieties and all of the Z moieties are independently Alk. In another embodiment, all of the Y moieties are independently Alk and none of the Z moieties are Alk.

Alk may have 1 to 30, in particular 2 to 24, carbon atoms. Alk may be optionally substituted. Alk may be unsubstituted. Alk may be linear or branched.

Examples of suitable Alk include methyl, propyl, 1-methylethyl, butyl, S₁₋₂-methylpropyl, pentyl, S₁₋₃-methylbutyl, hexyl, S₁₋₄-methylpentyl, heptyl, S₁₋₅-methylhexyl, octyl, S₁₋₆-methylheptyl, 2-ethylhexyl, nonyl, S₁₋₇-methyloctyl, decyl, S₁₋₈-methylnonyl, undecyl, S₁₋₉-methyldecyl, dodecyl, S₁₋₁₀-methylundecyl, tridecyl, S₁₋₁₁-methyldodecyl, tetradecyl, S₁₋₁₂-methyltridecyl, pentadecyl, S₁₋₁₃-methyltetradecyl, hexadecyl, S₁₋₁₄-methylpentadecyl, heptadecyl, S₁₋₁₅-methylhexadecyl, octadecyl, S₁₋₁₆-methylheptadecyl, nonadecyl, S₁₋₁₇-methyloctadecyl, icosyl, S₁₋₁₈-methylnonadecyl, henicosyl, S₁₋₁₉-methylicosyl, docosyl, S₁₋₂₀-methylhenicosyl, 2-propylheptyl, 2-propylnonyl, 2-pentylnonyl, 2-butyloctyl, 2-butyldecyl, 2-hexyloctyl, 2-hexyldecyl, 2-octyldecyl, 2-hexyldodecyl, 2-octyldodecyl, 2-decyltetradecyl, 6-methyldodecyl and isomers thereof, wherein S_{a-b} represents an integer that may be of any value ranging from a to b, S_{a-b} indicating the position of the substituent on the alkyl. Group "S₁₋₁₁-methyldodecyl" corresponds to a dodecyl group substituted by a methyl group in position 1, 2, 3, 4, 5, 6, 7, 8, 9, 10 or 11. By isomers, it is meant an alkyl group comprising the same number of carbon atoms but having a different substitution pattern, for example an ethyl substituent instead of a methyl substituent or a higher number of methyl substituents. Accordingly, a 2-ethylhexyl group is an isomer of a 6-methylheptyl group. The aforementioned alkyl groups may be linked to the X₁ or X₂ group in position 1. Accordingly, a 2-ethylhexyl group may be represented by the following formula: 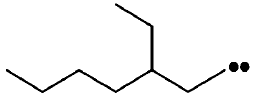 wherein symbol •• represents a point of attachment to X₁ or X₂.

In particular, part or all of the Y moieties and/or part of all of the Z moieties may independently be Alk selected from ethyl, propyl, 1-methylethyl, butyl, S₁₋₂-methylpropyl, pentyl, S₁₋₃-methylbutyl, hexyl, S₁₋₄-methylpentyl, heptyl, S₁₋₅-methylhexyl, octyl, S₁₋₆-methylheptyl, 2-ethylhexyl and isomers thereof.

Part or all of the Y moieties and/or part of all of the Z moieties may independently be an alkoxylated alkyl. When Y is an alkoxylated alkyl X₁ is preferably O. When Z is an alkoxylated alkyl X₂ is preferably O. In one embodiment, all of the Y moieties and all of the Z moieties are independently an alkoxylated alkyl. In another embodiment, all of the Y moieties are independently an alkoxylated alkyl and none of the Z moieties are an alkoxylated alkyl.

The alkoxylated alkyl may correspond to a group of formula ••-[(CR_{g}R'_{g})_{g}-O]ₕ-Alk wherein
Alk is as defined above;
R_{g}, and R'_{g}, are independently H or alkyl, in particular H or methyl, more particularly H;
g is 2 to 4, in particular 2;
h is 1 to 30, in particular 1 to 20, more particularly 1 to 10;
symbol •• represents a point of attachment to X₁ or X₂.

Part or all of the Y moieties and/or part of all of the Z moieties may form a polymeric backbone.

In one embodiment, the Y moieties form a polymeric backbone. In particular, the compound of formula (I) may correspond to a polymeric backbone having side groups (i.e. pendant groups) comprising photoinitiator moieties. For example, the compound of formula I may be represented by the following formula: 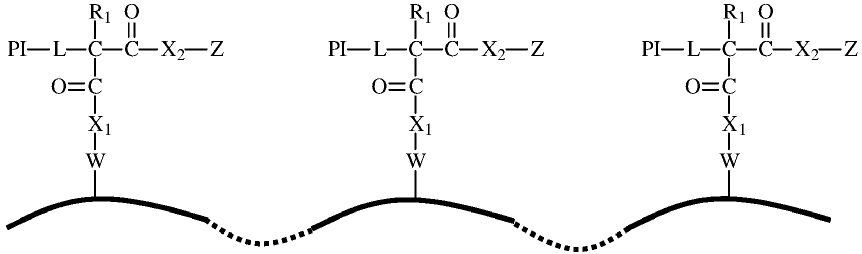 wherein
PI, L, R₁, X₁, X₂, and Z are as defined above;
W is a bond or a divalent linker.

In another embodiment, the Y and Z moieties may form a polymeric backbone. In particular, the compound of formula I may correspond to a polymeric backbone with (in-chain) repeating units comprising a photoinitiator moiety. For example, the compound of formula I may be represented by the following formula: 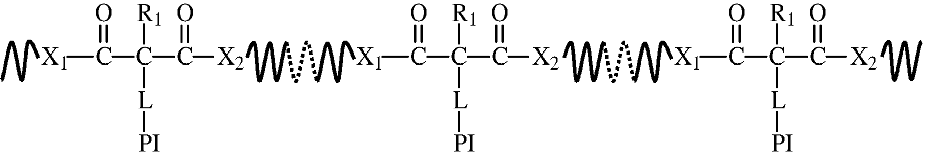 wherein PI, L, R₁, X₁ and X₂ are as defined above.

Part or all of the Y moieties and/or part of all of the Z moieties may independently be a polymeric core. In one embodiment, the Y moieties form a polymeric core. The polymeric core may be a star shaped, branched or dendritic polymeric core, preferably a polyether or polyester core. Dendritic polymeric cores include dendrimers, dendrons, dendrigrafts and hyperbranched polymeric cores.

Part or all of the Y moieties and/or part of all of the Z moieties may be a group of formula (IX): 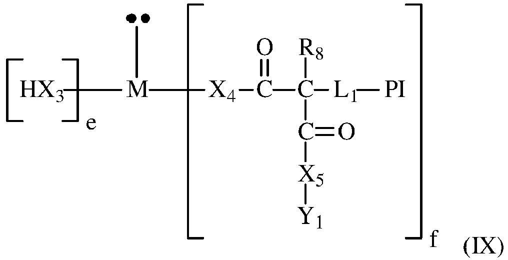 wherein
M is a (e+f)valent linker;
each PI may independently be a photoinitiator moiety comprising an arylketone as defined above;
each L₁ may independently be as defined above for linker L;
each R₈ may independently be as defined above for R₁;
each X₃, X₄ and X₅ may independently be as defined above for X₁ and X₂;
each R₉ may independently be as defined above for R₂;
each Y₁ is independently H, an optionally substituted alkyl, an alkoxylated alkyl or Y₁ may form a ring with Y, Z or another Y₁;
e is from 0 to 6;
f is from 0 to 6;
with the proviso that at least one of e and f is not 0;
symbol •• represents a point of attachment to X₁ or X₂.

M is an (e+f)valent linker. In particular, M may be a divalent, trivalent, tetravalent, pentavalent or hexavalent linker.

M may be an aromatic, aliphatic or cycloaliphatic hydrocarbon linker, an isocyanurate linker, a polyether linker, a polyamine linker, a polysulfide linker, a polyester linker, a polycarbonate linker, a polycaprolactone linker, a polyurethane linker, a polyorganosiloxane linker, a polybutadiene linker, and combinations thereof. In particular, M may be selected from an aromatic, aliphatic or cycloaliphatic hydrocarbon linker, a polyether linker, a polyester linker and combinations thereof.

When X₃ is O, M may be the residue of a polyol P_{OH} without the OH groups. When X₃ is NR₂, M may be the residue of a polyamine P_{NH} without the NR₂ groups. When X₃ is S, M may be the residue of a polythiol P_{SH} without the SH groups.

Examples of suitable polyols P_{OH} include ethylene glycol, 1,2- or 1,3-propylene glycol, 1,2-, 1,3- or 1,4-butylene glycol, 1,5-pentanediol, 1,6-hexanediol, 1,8-octanediol, 1,9-nonanediol, 1,10-decanediol, 1,12-dodecanediol, 2-methyl-1,3-propanediol, 2,2-diethyl-1,3-propanediol, 3-methyl-1,5-pentanediol, 3,3-dimethyl-1,5-pentanediol, neopentyl glycol, 2,4-diethyl-1,5-pentanediol, cyclohexanediol, cyclohexane-1,4-dimethanol, norbornene dimethanol, norbornane dimethanol, tricyclodecanediol, tricyclodecane dimethanol, bisphenol A, B, F or S, hydrogenated bisphenol A, B, F or S, trimethylolmethane, trimethylolethane, trimethylolpropane, di(trimethylolpropane), triethylolpropane, pentaerythritol, di(pentaerythritol), glycerol, di-, tri- or tetraglycerol, polyglycerol, di-, tri- or tetraethylene glycol, di-, tri- or tetrapropylene glycol, di-, tri- or tetrabutylene glycol, a polyethylene glycol, a polypropylene glycol, a polytetramethylene glycol, a poly(ethylene glycol-co-propylene glycol), a sugar alcohol, a dianhydrohexitol (i.e. isosorbide, isomannide, isoidide), tris(2-hydroxyethyl)isocyanurate, a polybutadiene polyol, a polyester polyol, a polyether polyol, a polyorganosiloxane polyol, a polycarbonate polyol as well as the alkoxylated (e.g., ethoxylated and/or propoxylated) derivatives thereof and the derivatives obtained by ring-opening polymerization of ε-caprolactone initiated with one of the aforementioned polyols.

Examples of suitable polyamines P_{NH} include polyether polyamines piperazine, meta- or para-phenylenediamine, meta- or para-xylylenediamine, meta- or para-toluylene diamine, 3,4'-diaminodiphenylether, 4,4'-diaminodiphenylether, 4,4'-diaminodiphenylmethane, diethylene triamine, dipropylene triamine, triethylene tetramine, 3,3'-diamino-*N*-methyldipropylamine, poly(ethylene imine) and poly(propylene imine). Polyether polyamines comprise two or three primary amino groups (-NH₂) and a polyether linker connecting the amino groups. The polyether linker may comprise 1 to 200, in particular 5 to 100, oxyalkylene units. Such compounds are available under reference Jeffamine^{®} Series D, ED, EDR and T from Hunstmann.

Examples of suitable polythiols P_{SH} include 1,2-ethanedithiol, 1,2- or 1,3-propanedithiol, 1,3-, 2,3- or 1,4-butanedithiol, 1,3- or 1,5-pentanedithiol, 1,6-hexanedithiol, 1,3-dimercapto-3-methylbutane, dipentenedimercaptan, ethylcyclohexyldithiol (ECHDT), bis(2-mercaptoethyl)sulfide (DMDS), 1,8-dimercapto-3,6-dioxaoctane (DMDO), 1,5-dimercapto-3-oxapentane, 2,5-dimercaptomethyl-1,4-dithiane, dimercaptoethylsulfide, pentaerythritol tetrakis(3-mercaptopropionate), pentaerythritol tetrakis(2-mercaptoacetate), trimethylolpropane tris(3-mercaptopropionate), trimethylolpropane tris(2-mercaptoacetate), 4-mercaptomethyl-3,6-dithia-1,8-octanedithiol, 4-tert-butyl-1,2-benzenedithiol, 4,4'-thiodibenzenethiol, , benzenedithiol, ethylene glycol di(2-mercaptoacetate), ethylene glycol di(3-mercaptopropionate), poly(ethylene glycol) di(2-mercaptoacetate) and poly(ethylene glycol) di(3-mercaptopropionate).

In one embodiment, M may be a divalent linker selected from one of formula (D1)-(D5):

-(CRᵢR'ᵢ)ᵢ- (D1)

-[(CRⱼR'ⱼ)ⱼ-O]k-(CRⱼR'ⱼ)ⱼ- (D2)

-[(CRₖR'ₖ)_{j'}-O]_{k'}-(CR₁R'₁)¡'-[O-(CRₖR'ₖ)_{j"}]_{k"}- (D3)

-[(CRₘR'ₘ)₁-N(Rₙ)]ₘ-(CRₘR'ₘ)₁- (D4)

-[(CRₒR'o)_{1'}-C(=O)O]m_{'}-[(CRⱼR'ⱼ)ⱼ-O]k*-(CRⱼR'ⱼ)ⱼ-[OC(=O)-(CRₒR'ₒ)₁"]ₘ"- (D5);

Rᵢ, R'ᵢ, Rⱼ, R'ⱼ, Rₖ, R'ₖ, R₁, R'₁, Rₘ ,R'ₘ, Rₙ, Rₒ and R'ₒ are independently H or alkyl, in particular H, methyl or ethyl;
i and i' are independently 2 to 20;
j, j', j" and 1 are independently 2 to 4;
k and m are independently 1 to 20 ;
k' and k" are independently 0 to 20 with the proviso that at least one of k' and k" is not 0;
k* is 0 to 20;
1 and 1" are independently 3 to 12;
m' and m" are independently 0 to 20 with the proviso that at least one of m' and m" is not 0.

In particular, M may be a divalent linker selected from an alkylene such as ethylene, 1,2- or 1,3-propanediyl, 1,2-, 1,3-or 1,4-butanediyl, 1,5-pentanediyl, 1,6-hexanediyl, 1,8-octanediyl, 1,9-nonanediyl, 1,10-decanediyl, 1,12-decanediyl, 2-methyl-1,3-propanediyl, 2,2-diethyl-1,3-propanediyl, 3-methyl-1,5-pentanediyl, 3,3-dimethyl-1,5-pentanediyl, 2,2-dimethyl-1,3-propanediyl, 2,4-diethyl-1,5-pentanediyl; an alkoxylated (in particular an ethoxylated and/or propoxylated) derivative of the aforementioned alkylenes; an esterified (in particular by ring-opening polymerization of a lactone such as ε-caprolactone) derivative of the aforementioned alkylenes; a residue of a di-, tri-, tetra- or polyoxyalkene without the OH groups such as di-, tri- or tetraethylene glycol, di-, tri- or tetrapropylene glycol, di-, tri- or tetrabutylene glycol, polyethylene glycol, polypropylene glycol, polytetramethylene glycol and poly(ethylene glycol-co-propylene glycol).

In one embodiment, M may be a trivalent linker corresponding to formula (X), a tetravalent linker corresponding to formula (XI) or (XII) or an hexavalent linker corresponding to formula (XIII): 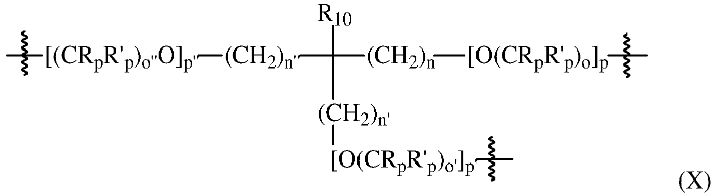 wherein
Rₚ and R'ₚ are independently H or methyl;
R₁₀ is selected from H, alkyl and alkoxy, in particular R₁₀ is alkyl;
n, n' and n" are independently 0 to 2 with the proviso that at least 2 among n, n' and n" are not 0, in particular n, n' and n" are all 1 or n is 0 and n' and n" are 1;
o, o' and o" are independently 2 to 4, in particular 2;
p, p' and p" are independently 0 to 10, in particular 0 to 6; 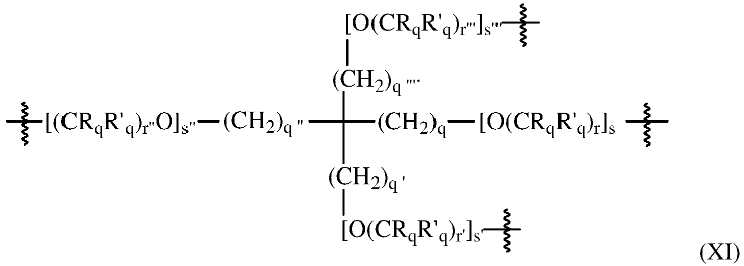
wherein
R_{q} and R'_{q} are independently H or methyl;
q, q', q" and q'" are independently 0 to 2 with the proviso that at least 3 among q, q', q" and
q'" are not 0, in particular q, q', q" and q'" are all 1,
r, r', r" and r'" are independently 2 to 4, in particular 2;
s, s', s" and s'" are independently 0 to 10, in particular 1 to 6; 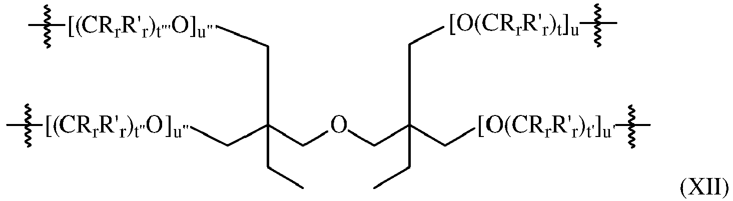
wherein
Rᵣ and R'r are independently H or methyl;
t, t', t" and t'" are independently 2 to 4, in particular 2;
u, u', u" and u'" are independently 0 to 10, in particular 1 to 6; 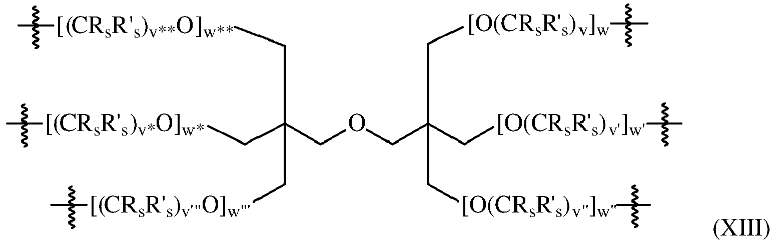
Rₛ and R'ₛ are independently H or methyl;
v, v', v", v'", v* and v** are independently 2 to 4, in particular 2;
w, w', w", w"', w* and w** are independently 0 to 10, in particular 1 to 6.

In one embodiment, Y₁ may form a ring with Y, Z or another Y₁. Accordingly, the compound of the invention may correspond to the following formula (Ia) or (Ib): 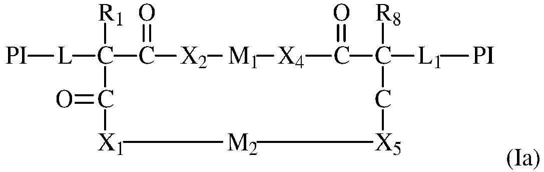 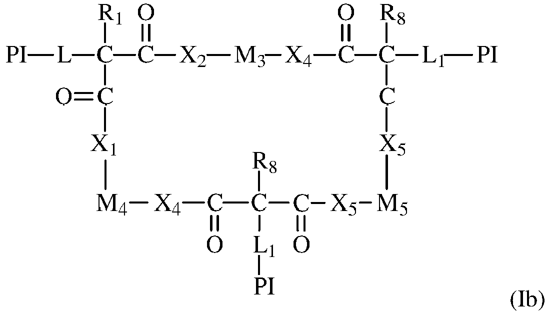
wherein PI, L, L₁, R₁, R₈, X₁, X₂, X₄ and X₅ are as defined above;
M₃, M₄ and M₅ are independently an organic linker.

In particular, M₃, M₄ and M₅ may independently be an aromatic, aliphatic or cycloaliphatic hydrocarbon linker, a polyether linker, a polyamine linker, a polysulfide linker, a polyester linker, a polycaprolactone linker, a polyurethane linker, a polydimethylsiloxane linker, a polybutadiene linker, and combinations thereof. In particular, M₃, M₄ and M₅ may independently be selected from an aromatic, aliphatic or cycloaliphatic hydrocarbon linker, a polyether linker, a polyester linker and combinations thereof.

When X₁, X₂, X₄ and X₅ are O, M₃, M₄ and M₅ may independently be the residue of a polyol P_{OH} without the OH groups as defined above for M. When X₁, X₂, X₄ and X₅ are NR₂, M₃, M₄ and M₅ may independently be the residue of a polyamine P_{NH} without the NR₂ groups as defined above for M. When X₁, X₂, X₄ and X₅ are S, M₃, M₄ and M₅ may independently be the residue of a polythiol P_{SH} without the SH groups as defined above for M.

In particular, M₃, M₄ and M₅ may independently be a divalent linker as defined above for M (formula (D1)-(D5) and the specific examples of divalent linkers).

In one embodiment, Y may form a ring with Z. Accordingly, the compound of the invention may correspond to the following formula (Ic) 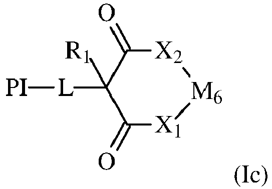
wherein PI, L, R₁, X₁ and X₂ are as defined above;
M₆ is an organic linker.

In particular, M₆ may be an aromatic, aliphatic or cycloaliphatic hydrocarbon linker, a polyether linker, a polyamine linker, a polysulfide linker, a polyester linker, a polycaprolactone linker, a polyurethane linker, a polydimethylsiloxane linker, a polybutadiene linker, and combinations thereof. In particular, M₆ may be selected from an aromatic, aliphatic or cycloaliphatic hydrocarbon linker, a polyether linker, a polyester linker and combinations thereof.

When X₁ and X₂ are O, M₆ may be the residue of a polyol P_{OH} without the OH groups as defined above for M. When X₁ and X₂ are NR₂, M₆ may be the residue of a polyamine P_{NH} without the NR₂ groups as defined above for M. When X₁ and X₂ are S, M₆ may be the residue of a polythiol P_{OH} without the SH groups as defined above for M.

In particular, M₆ may be a divalent linker as defined above for M (formula (D1)-(D5) and the specific examples of divalent linkers).

In one embodiment, PI, L, R₁, X₁ and X₂ are as defined above and Y and Z are independently an alkyl as defined above. The alkyls may be the same or different.

In another embodiment, PI, L, R₁, X₁ and X₂ are as defined above and Y and Z are independently an alkoxylated alkyl as defined above. The alkoxylated alkyls may be the same or different.

In another embodiment, PI, L, R₁, X₁ and X₂ are as defined above, Y is an alkyl and Z is an alkoxylated alkyl as defined above.

In another embodiment, PI, L, R₁, X₁ and X₂ are as defined above, Y is H and Z is an alkyl or an alkoxylated alkyl as defined above.

In another embodiment, PI, L, R₁, X₁ and X₂ are as defined above and Y and Z are independently a group of formula (IX) as defined above. The groups of formula (IX) may be the same or different.

In another embodiment, PI, L, R₁, X₁ and X₂ are as defined above, Y is H, an alkyl or an alkoxylated alkyl as defined above and Z is a group of formula (IX) as defined above.

In another embodiment, PI, L, R₁, X₁ and X₂ are as defined above, Y is an H, an alkyl or an alkoxylated alkyl as defined above and Z forms a polymeric backbone or a polymeric core as defined above.

In another embodiment, PI, L, R₁, X₁ and X₂ are as defined above, and Y and Z form a polymeric backbone as defined above.

In another embodiment, PI, L, R₁, X₁ and X₂ are as defined above, and Y and Z form a ring as defined above.

The compound of formula (I) of the invention may advantageously be liquid at 20°C. Alternatively, the compound of formula (I) may be soluble at 25°C in one or more substances conventionally used in curable compositions, in particular in a non-reactive solvent, a (meth)acrylate monomer, a (meth)acrylate oligomer and mixtures thereof. Such substances are described in detail hereinafter. The term "compound A is soluble at 25°C in a substance B" means that, at 25°C compound A is soluble (fully dissolved) in a composition comprising more than 5%, more than 10%, more than 15%, more than 20%, more than 25%, more than 30% or more than 35% by weight of compound A based on the total weight of compound A and substance B. Advantageously, compound A may remain solubilized (fully dissolved, no precipitation or crystallization) in one or more substances conventionally used in curable compositions for at least 24h, in particular at least 48h, more particularly for at least one week.

Compounds of formula (I) may be obtained by conventional acylation reactions. A compound of formula (XIV) having a benzophenone moiety may be obtained by reacting an acyl halide (such as an acyl choride) of formula (XV) with a substituted phenyl of formula (XVI) in the presence of a Lewis acid (such as a metal halide, for example iron chloride or aluminum chloride) according to the following scheme: 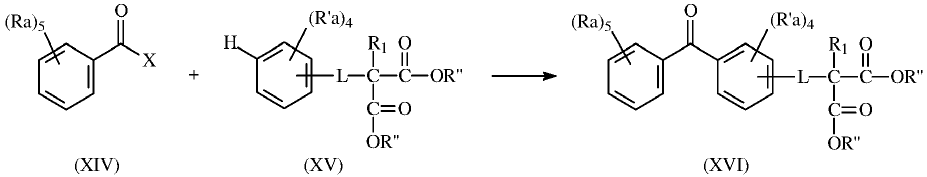
wherein L, R₁, Rₐ and R'ₐ are as defined herein;
X is a halogen atom, in particular Cl; and
R" is H or alkyl, in particular methyl or ethyl.

A compound of formula (XIX) having a thioxanthone, anthrone or anthraquinone moiety may be obtained by reacting an acyl halide of formula (XVII) with a substituted phenyl of formula (XVIII) in the presence of a Lewis acid (such as a metal halide, for example iron chloride or aluminum chloride) according to the following scheme: 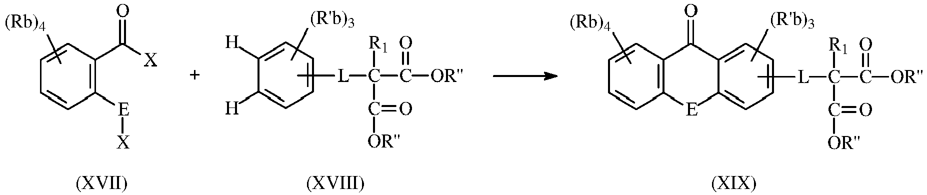
wherein E is S, C(=O) or CRoR'o;
L, R₀, R'₀, R₁, R_{b} and R'_{b} are as defined herein;
X is a halogen atom, in particular Cl; and
R" is H or alkyl, in particular methyl or ethyl.

Compounds of formula (I) may also be synthesized according to the scheme below by reacting malonate esters of formula (XXI) with haloalkyl- or acyl halide-substituted benzophenones, thioxanthones, anthraquinones, xanthones, anthrones or acridones of formula (XX) wherein L is alkylene or C=O, in the presence of a suitable base (e.g. sodium or potassium hydroxide, sodium or potassium carbonate, calcium oxide). Direct cross-coupling of malonate esters of formula (XXI) with halo-substituted benzophenones, thioxanthones, anthraquinones, xanthones, anthrones or acridones of formula (XX) wherein L is bond may also be possible with the aid of a transition metal catalyst (e.g. a palladium catalyst). 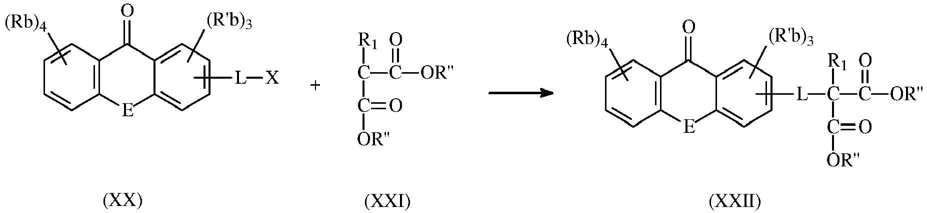
wherein E is absent or as defined herein;
L, R₁, R_{b} and R'_{b} are as defined herein;
X is a halogen atom, in particular Cl; and
R" is H or alkyl, in particular methyl or ethyl.

Haloalkyl-substituted photoinitiators of formula (XX) wherein L is alkylene may be prepared by the halogenation of alkyl-substituted photoinitiators, for instance, by using free-radical halogenation. For example, bromomethyl-substituted photoinitiators can be prepared by the radical bromination of methyl-substituted photoinitiators in the presence of a bromine source such as *N*-bromosuccinimide, and a radical initiator such as benzoyl peroxide.

Acyl halide-substituted photoinitiators of formula (XX) wherein L is C(=O) may be prepared by the reaction of carboxylic acid-functionalized photoinitiators with suitable halogenating agents such as thionyl chloride.

The OR'' groups in the compounds of formula (XVI), (XIX) or (XXII) can then be converted into X₁-Y and/or X₂-Z groups with conventional reactions such as esterification/transesterification (conversion to O-Y and O-Z by reaction with alcohols HO-Y and/or HO-Z), amidation (conversion to NR₂-Y and NR₂-Z by reaction with amines HNR₂-Y and/or HNR₂-Z) or thioesterification (conversion to S-Y and S-Z by reaction with thiols HS-Y and/or HS-Z) wherein X₁, X₂, Y and Z are as defined herein.

### Photoinitiator composition

The photoinitiator composition of the invention comprises a mixture of photoinitiators.

The photoinitiator composition of the invention may comprise a mixture of at least two distinct compounds of formula (I) as defined above.

The photoinitiator composition of the invention may comprise a compound of formula (I) as defined above and a photoinitiator other than a compound of formula (I).

The photoinitiator other than a compound of formula (I) may be a radical photoinitiator, in particular a radical photoinitiator having Norrish type I activity and/or Norrish type II activity, more particularly a radical photoinitiator having Norrish type II activity.

Non-limiting types of radical photoinitiators suitable for use in the curable compositions of the present invention include, for example, benzoins, benzoin ethers, acetophenones, α-hydroxy acetophenones, benzyl, benzyl ketals, anthraquinones, phosphine oxides, acylphosphine oxides, α-hydroxyketones, phenylglyoxylates, α-aminoketones, benzophenones, thioxanthones, xanthones, acridine derivatives, phenazene derivatives, quinoxaline derivatives, triazine compounds, benzoyl formates, aromatic oximes, metallocenes, acylsilyl or acylgermanyl compounds, camphorquinones, polymeric derivatives thereof, and mixtures thereof.

Examples of suitable radical photoinitiators include, but are not limited to, 2-methylanthraquinone, 2-ethylanthraquinone, 2-chloroanthraquinone, 2-benzyanthraquinone, 2-t-butylanthraquinone, 1,2-benzo-9,10-anthraquinone, benzyl, benzoins, benzoin ethers, benzoin, benzoin methyl ether, benzoin ethyl ether, benzoin isopropyl ether, alpha-methylbenzoin, alpha-phenylbenzoin, Michler's ketone, acetophenones such as 2,2-dialkoxybenzophenones and 1-hydroxyphenyl ketones, benzophenone, 4,4'-bis-(diethylamino) benzophenone, acetophenone, 2,2-diethyloxyacetophenone, diethyloxyacetophenone, 2-isopropylthioxanthone, thioxanthone, diethyl thioxanthone, 1,5-acetonaphthylene, benzil ketone, α-hydroxy keto, 2,4,6-trimethylbenzoyldiphenyl phosphine oxide, benzyl dimethyl ketal, 2,2-dimethoxy-1,2-diphenylethanone, 1-hydroxycylclohexyl phenyl ketone, 2-methyl-1-[4-(methylthio) phenyl]-2-morpholinopropanone-1, 2-hydroxy-2-methyl-1-phenyl-propanone, oligomeric α-hydroxy ketone, benzoyl phosphine oxides, phenylbis(2,4,6-trimethylbenzoyl)phosphine oxide, ethyl(2,4,6-trimethylbenzoyl)phenyl phosphinate, anisoin, anthraquinone, anthraquinone-2-sulfonic acid, sodium salt monohydrate, (benzene) tricarbonylchromium, benzil, benzoin isobutyl ether, benzophenone/1-hydroxycyclohexyl phenyl ketone, 50/50 blend, 3,3',4,4'-benzophenonetetracarboxylic dianhydride, 4-benzoylbiphenyl, 2-benzyl-2-(dimethylamino)-4'-morpholinobutyrophenone, 4,4'-bis(diethylamino)benzophenone, 4,4'-bis(dimethylamino)benzophenone, camphorquinone, 2-chlorothioxanthen-9-one, dibenzosuberenone, 4,4'-dihydroxybenzophenone, 2,2-dimethoxy-2-phenylacetophenone, 4-(dimethylamino)benzophenone, 4,4'-dimethylbenzil, 2,5-dimethylbenzophenone, 3,4-dimethylbenzophenone, diphenyl(2,4,6-trimethylbenzoyl)phosphine oxide /2-hydroxy-2-methylpropiophenone, 50/50 blend, 4'-ethoxyacetophenone, 2,4,6-trimethylbenzoyldiphenylphophine oxide, phenyl bis(2,4,6-trimethyl benzoyl)phosphine oxide, ferrocene, 3'-hydroxyacetophenone, 4'-hydroxyacetophenone, 3-hydroxybenzophenone, 4-hydroxybenzophenone, 1-hydroxycyclohexyl phenyl ketone, 2-hydroxy-2-methylpropiophenone, 2-methylbenzophenone, 3-methylbenzophenone, methybenzoylformate, 2-methyl-4'-(methylthio)-2-morpholinopropiophenone, phenanthrenequinone, 4'-phenoxyacetophenone, (cumene)cyclopentadienyl iron(ii) hexafluorophosphate, 9,10-diethoxy and 9,10-dibutoxyanthracene, 2-ethyl-9,10-dimethoxyanthracene, thioxanthen-9-one and combinations thereof.

In particular, the photoinitiator other a compound of formula (I) may be a benzophenone such as SpeedCure^{®} BP (benzophenone), SpeedCure^{®} 7005 (polymeric benzophenone) or SpeedCure^{®} 7006 (polymeric benzophenone); a thioxanthone such as SpeedCure^{®} 7010 (polymeric thioxanthone), SpeedCure^{®} ITX (isopropyl thioxanthone) or SpeedCure^{®} CPTX (1-chloro-4-propoxythioxanthone); an α-hydroxy acetophenone; an acylphosphine oxide such as SpeedCure^{®} BPO (phenyl bis(2,4,6-trimethylbenzoyl)-phosphine oxide), SpeedCure^{®} TPO (2,4,6-trimethylbenzoyldiphenylphosphine oxide) or SpeedCure^{®} TPO-L (ethyl (2,4,6-trimethylbenzoyl)phenyl phosphinate). Preferably, the photoinitiator other than a compound of formula (I) is Speedcure^{®} CPTX.

In another embodiment, the photoinitiator other than a compound of formula (I) may be a cationic photoinitiator. A cationic photoinitiator is advantageously present when the composition comprises a cationically polymerizable compound as defined below.

Suitable cationic photoinitiators include any type of photoinitiator that, upon exposure to radiation such as actinic radiation, forms cations (e.g., Brönsted or Lewis acids) that initiate the reaction of the monomeric and (if present) oligomeric cationically polymerizing organic substances in the curable composition. For example, a cationic photoinitiator may be comprised of a cationic portion and an anionic portion. The cationic portion of the photoinitiator molecule can be responsible for the absorption of UV radiation while the anionic portion of the molecule becomes a strong acid after UV absorption.

In particular, the photoinitiator other than a compound of formula (I) may be a cationic photoinitiator selected from onium salts with anions of weak nucleophilicity, such as halonium salts, iodonium salts (e.g., diaryliodonium salts such as bis(4-t-butylphenyl) iodonium perfluoro-1-butane sulfonate) or sulfonium salts (e.g., triarylsulfonium salts such as triarylsulfonium hexafluoroantimonate salts); sulfoxonium salts; diazonium salts; metallocene salts; and mixtures thereof.

The molar ratio between a compound of formula (I) and a photoinitiator other than a compound of formula (I) may be varied as may be appropriate depending on the photoinitiator(s) selected, the amounts and types of polymerizable species that are intended to be photopolymerized, the radiation source and the radiation conditions used, among other factors. Typically, however, the molar ratio between a compound of formula (I) and a photoinitiator other than a compound of formula (I) may be from 10/90 to 90/10, or from 20/80 to 80/20, or from 30/70 to 70/30, or from 40/60 to 60/40 or 45/55 to 55/45.

### Photopolymerization process

The compound of formula (I) as defined above or the photoinitiator compositions as defined above may be used in a photopolymerization process, i.e. a process for photopolymerizing (e.g. curing) one or more ethylenically unsaturated compounds.

The process for photopolymerizing one or more ethylenically unsaturated compounds comprises contacting one or more ethylenically unsaturated compounds with a compound of formula (I) as defined above or a photoinitiator composition as defined above, and irradiating the mixture, in particular with visible, near-UV and/or UV light. When the compound of formula (I) is a thioxanthone, the mixture may be irradiated with a LED light source, in particular a LED light source having an emission band in the range of 365-420 nm.

### Curable composition comprising an ethylenically unsaturated compound

The curable composition of the invention comprises a compound of formula (I) as defined above or a photoinitiator composition as defined above, referred to as component a). The curable composition of the invention further comprises an ethylenically unsaturated compound, referred to as component b).

The curable composition of the invention may comprise from 0.05% to 30%, from 0.1% to 20%, from 0.2 to 15%, from 0.5 to 10% or from 1 to 5%, by weight of compound of formula (I) based on the total weight of the ethylenically unsaturated compound.

The curable composition of the invention may comprise from 0.05% to 30%, from 0.1% to 20%, from 0.2 to 15%, from 0.5 to 10% or from 1 to 5%, by weight of photoinitiator other than a compound of formula (I) based on the total weight of the ethylenically unsaturated compound.

The curable composition of the invention may comprise:
- from 0.05 to 30%, from 0.1 to 20%, from 0.2 to 15%, from 0.5 to 10% or from 1 to 5% of component a);
- from 70 to 99,95%, from 80 to 99.9%, from 85 to 99.8%, from 90 to 99.5% or from 95 to 99% of component b);
   the % being % by weight based on the total weight of components a) and b).

The curable composition of the invention may further comprise one or more compounds selected from:
- an amine synergist;
- a cationically polymerizable compound;
- an additive;
- a solvent.

### Ethylenically unsaturated compound

The curable composition of the invention comprises an ethylenically unsaturated compound. The curable composition of the invention may comprise a mixture of ethylenically unsaturated compounds.

As used herein, the term "ethylenically unsaturated compound" means a compound that comprises a polymerizable carbon-carbon double bond. A polymerizable carbon-carbon double bond is a carbon-carbon double bond that can react with another carbon-carbon double bond in a polymerization reaction. A polymerizable carbon-carbon double bond is generally comprised in a group selected from acrylate (including cyanoacrylate), methacrylate, acrylamide, methacrylamide, styrene, maleate, fumarate, itaconate, allyl, propenyl, vinyl and combinations thereof, preferably selected from acrylate, methacrylate and vinyl, more preferably selected from acrylate and methacrylate. The carbon-carbon double bonds of a phenyl ring are not considered as polymerizable carbon-carbon double bonds.

In one embodiment, the ethylenically unsaturated compound may be selected from a (meth)acrylate-functionalized monomer, a (meth)acrylate-functionalized oligomer, an amine-modified acrylate and mixtures thereof.

The total amount of ethylenically unsaturated compound (including (meth)acrylate functionalized monomer, (meth)acrylate functionalized oligomer and amine-modified acrylate) in the curable composition may be from 40 to 99.5%, in particular 50 to 95%, more particularly 60 to 90%, by weight based on the weight of the composition. In particular, the curable composition may comprise 40 to 80%, or 40 to 75% or 40 to 70% or 40 to 65% or 40 to 60% by weight of ethylenically unsaturated compound based on the weight of the composition. Alternatively, the curable composition may comprise 60 to 99.5%, or 65 to 99.5% or 70 to 99.5% or 75 to 99.5% or 80 to 99.5% by weight of ethylenically unsaturated compound based on the weight of the composition.

As used herein, the term "(meth)acrylate-functionalized monomer" means a monomer comprising a (meth)acrylate group, in particular an acrylate group. The term "(meth)acrylate-functionalized oligomer" means an oligomer comprising a (meth)acrylate group, in particular an acrylate group. The term "(meth)acrylate group" encompasses acrylate groups (-O-CO-CH=CH₂) and methacrylate groups (-O-CO-C(CH₃)=CH₂).

In one embodiment, the ethylenically unsaturated compound comprises a (meth)acrylate-functionalized monomer. The ethylenically unsaturated compound may comprise a mixture of (meth)acrylate-functionalized monomers.

The (meth)acrylate-functionalized monomer may have a molecular weight of less than 600 g/mol, in particular from 100 to 550 g/mol, more particularly 200 to 500 g/mol.

The (meth)acrylate-functionalized monomer may have 1 to 6 (meth)acrylate groups, in particular 1 to 3 (meth)acrylate groups.

The (meth)acrylate-functionalized monomer may comprise a mixture of (meth)acrylate-functionalized monomers having different functionalities. For example the (meth)acrylate-functionalized monomer may comprise a mixture of a (meth)acrylate-functionalized monomer containing a single acrylate or methacrylate group per molecule (referred to herein as "mono(meth)acrylate-functionalized compounds") and a (meth)acrylate-functionalized monomer containing 2 or more, preferably 2 or 3, acrylate and/or methacrylate groups per molecule.

In one embodiment, the (meth)acrylate functionalized monomer comprises a mono(meth)acrylate-functionalized monomer. The mono(meth)acrylate-functionalized monomer may advantageously function as a reactive diluent and reduce the viscosity of the composition of the invention.

Examples of suitable mono(meth)acrylate-functionalized monomers include, but are not limited to, mono-(meth)acrylate esters of aliphatic alcohols (wherein the aliphatic alcohol may be straight chain, branched or alicyclic and may be a mono-alcohol, a di-alcohol or a polyalcohol, provided only one hydroxyl group is esterified with (meth)acrylic acid); mono-(meth)acrylate esters of aromatic alcohols (such as phenols, including alkylated phenols); mono-(meth)acrylate esters of alkylaryl alcohols (such as benzyl alcohol); mono-(meth)acrylate esters of oligomeric and polymeric glycols such as diethylene glycol, triethylene glycol, dipropylene glycol, tripropylene glycol, polyethylene glycol, and polypropylene glycol); mono-(meth)acrylate esters of monoalkyl ethers of glycols and oligoglycols; mono-(meth)acrylate esters of alkoxylated (e.g., ethoxylated and/or propoxylated) aliphatic alcohols (wherein the aliphatic alcohol may be straight chain, branched or alicyclic and may be a mono-alcohol, a di-alcohol or a polyalcohol, provided only one hydroxyl group of the alkoxylated aliphatic alcohol is esterified with (meth)acrylic acid); mono-(meth)acrylate esters of alkoxylated (e.g., ethoxylated and/or propoxylated) aromatic alcohols (such as alkoxylated phenols); caprolactone mono(meth)acrylates; and the like.

The following compounds are specific examples of mono(meth)acrylate-functionalized monomers suitable for use in the curable compositions of the present invention: methyl (meth)acrylate; ethyl (meth)acrylate; n-propyl (meth)acrylate; n-butyl (meth)acrylate; isobutyl (meth)acrylate; n-hexyl (meth)acrylate; 2-ethylhexyl (meth)acrylate; n-octyl (meth)acrylate; isooctyl (meth)acrylate; n-decyl (meth)acrylate; n-dodecyl (meth)acrylate; tridecyl (meth)acrylate; tetradecyl (meth)acrylate; hexadecyl (meth)acrylate; 2-hydroxyethyl (meth)acrylate; 2- and 3-hydroxypropyl (meth)acrylate; 2-methoxyethyl (meth)acrylate; 2-ethoxyethyl (meth)acrylate; 2- and 3-ethoxypropyl (meth)acrylate; tetrahydrofurfuryl (meth)acrylate; alkoxylated tetrahydrofurfuryl (meth)acrylate; 2-(2-ethoxyethoxy)ethyl (meth)acrylate; cyclohexyl (meth)acrylate; glycidyl (meth)acrylate; isodecyl (meth)acrylate; lauryl (meth)acrylate; 2-phenoxyethyl (meth)acrylate; alkoxylated phenol (meth)acrylates; alkoxylated nonylphenol (meth)acrylates; cyclic trimethylolpropane formal (meth)acrylate; isobornyl (meth)acrylate; tricyclodecanemethanol (meth)acrylate; tert-butylcyclohexanol (meth)acrylate; trimethylcyclohexanol (meth)acrylate; diethylene glycol monomethyl ether (meth)acrylate; diethylene glycol monoethyl ether (meth)acrylate; diethylene glycol monobutyl ether (meth)acrylate; triethylene glycol monoethyl ether (meth)acrylate; ethoxylated lauryl (meth)acrylate; methoxy polyethylene glycol (meth)acrylates; hydroxyl ethyl-butyl urethane (meth)acrylates; 3-(2-hydroxyalkyl)oxazolidinone (meth)acrylates; and combinations thereof.

In one embodiment, the (meth)acrylate functionalized monomer may comprise a (meth)acrylate-functionalized monomer containing two or more (meth)acrylate groups per molecule.

Examples of suitable (meth)acrylate-functionalized monomers containing two or more (meth)acrylate groups per molecule include acrylate and methacrylate esters of polyhydric alcohols (organic compounds containing two or more, e.g., 2 to 6, hydroxyl groups per molecule). Specific examples of suitable polyhydric alcohols include C₂₋₂₀ alkylene glycols (glycols having a C₂₋₁₀ alkylene group may be preferred, in which the carbon chain may be branched; e.g., ethylene glycol, trimethylene glycol, 1,2-propylene glycol, 1,2-butanediol, 1,3-butanediol, 2,3-butanediol, tetramethylene glycol (1,4-butanediol), 1,5-pentanediol, 1,6-hexanediol, 1,8-octanediol, 1,9-nonanediol, 1,12-dodecanediol, cyclohexane-1,4-dimethanol, bisphenols, and hydrogenated bisphenols, as well as alkoxylated (e.g., ethoxylated and/or propoxylated) derivatives thereof), diethylene glycol, glycerin, alkoxylated glycerin, triethylene glycol, dipropylene glycol, tripropylene glycol, trimethylolpropane, alkoxylated trimethylolpropane, ditrimethylolpropane, alkoxylated ditrimethylolpropane, pentaerythritol, alkoxylated pentaerythritol, dipentaerythritol, alkoxylated dipentaerythritol, cyclohexanediol, alkoxylated cyclohexanediol, cyclohexanedimethanol, alkoxylated cyclohexanedimethanol, norbornene dimethanol, alkoxylated norbornene dimethanol, norbornane dimethanol, alkoxylated norbornane dimethanol, polyols containing an aromatic ring, cyclohexane-1,4-dimethanol ethylene oxide adducts, bis-phenol ethylene oxide adducts, hydrogenated bisphenol ethylene oxide adducts, bisphenol propylene oxide adducts, hydrogenated bisphenol propylene oxide adducts, cyclohexane-1,4-dimethanol propylene oxide adducts, sugar alcohols and alkoxylated sugar alcohols. Such polyhydric alcohols may be fully or partially esterified (with (meth)acrylic acid, (meth)acrylic anhydride, (meth)acryloyl chloride or the like), provided they contain at least two (meth)acrylate functional groups per molecule.

Exemplary (meth)acrylate-functionalized monomers containing two or more (meth)acrylate groups per molecule may include ethoxylated bisphenol A di(meth)acrylates; triethylene glycol di(meth)acrylate; ethylene glycol di(meth)acrylate; tetraethylene glycol di(meth)acrylate; polyethylene glycol di(meth)acrylates; 1,4-butanediol diacrylate; 1,4-butanediol dimethacrylate; diethylene glycol diacrylate; diethylene glycol dimethacrylate, 1,6-hexanediol diacrylate; 1,6-hexanediol dimethacrylate; neopentyl glycol diacrylate; neopentyl glycol di(meth)acrylate; polyethylene glycol (600) dimethacrylate (where 600 refers to the approximate number average molecular weight of the polyethylene glycol portion); polyethylene glycol (200) diacrylate; 1,12-dodecanediol dimethacrylate; tetraethylene glycol diacrylate; triethylene glycol diacrylate, 1,3-butylene glycol dimethacrylate, tripropylene glycol diacrylate, polybutadiene diacrylate; methyl pentanediol diacrylate; polyethylene glycol (400) diacrylate; ethoxylated₂ bisphenol A dimethacrylate; ethoxylated₃ bisphenol A dimethacrylate; ethoxylated₃ bisphenol A diacrylate; cyclohexane dimethanol dimethacrylate; cyclohexane dimethanol diacrylate; ethoxylated₁₀ bisphenol A dimethacrylate (where the numeral following "ethoxylated" is the average number of oxyalkylene moieties per molecule); dipropylene glycol diacrylate; ethoxylated₄ bisphenol A dimethacrylate; ethoxylated₆ bisphenol A dimethacrylate; ethoxylated₈ bisphenol A dimethacrylate; alkoxylated hexanediol diacrylates; alkoxylated cyclohexane dimethanol diacrylate; dodecane diacrylate; ethoxylated₄ bisphenol A diacrylate; ethoxylated₁₀ bisphenol A diacrylate; polyethylene glycol (400) dimethacrylate; polypropylene glycol (400) dimethacrylate; metallic diacrylates; modified metallic diacrylates; metallic dimethacrylates; polyethylene glycol (1000) dimethacrylate; methacrylated polybutadiene; propoxylated₂ neopentyl glycol diacrylate; ethoxylated₃₀ bisphenol A dimethacrylate; ethoxylated₃₀ bisphenol A diacrylate; alkoxylated neopentyl glycol diacrylates; polyethylene glycol dimethacrylates; 1,3-butylene glycol diacrylate; ethoxylated₂ bisphenol A dimethacrylate; dipropylene glycol diacrylate; ethoxylated₄ bisphenol A diacrylate; polyethylene glycol (600) diacrylate; polyethylene glycol (1000) dimethacrylate; tricyclodecane dimethanol diacrylate; propoxylated neopentyl glycol diacrylates such as propoxylated₂ neopentyl glycol diacrylate; diacrylates of alkoxylated aliphatic alcohols; trimethylolpropane trimethacrylate; trimethylolpropane triacrylate; tris (2-hydroxyethyl) isocyanurate triacrylate; ethoxylated₂₀ trimethylolpropane triacrylate; pentaerythritol triacrylate; ethoxylated₃ trimethylolpropane triacrylate; propoxylated₃ trimethylolpropane triacrylate; ethoxylated₆ trimethylolpropane triacrylate; propoxylated₆ trimethylolpropane triacrylate; ethoxylated₉ trimethylolpropane triacrylate; alkoxylated trifunctional acrylate esters; trifunctional methacrylate esters; trifunctional acrylate esters; propoxylated₃ glyceryl triacrylate; propoxylated_{5.5} glyceryl triacrylate; ethoxylated₁₅ trimethylolpropane triacrylate; trifunctional phosphoric acid esters; trifunctional acrylic acid esters; pentaerythritol tetraacrylate; di-trimethylolpropane tetraacrylate; ethoxylated₄ pentaerythritol tetraacrylate; pentaerythritol polyoxyethylene tetraacrylate; dipentaerythritol pentaacrylate; and pentaacrylate esters.

The curable composition of the invention may comprise 0 to 99.5%, in particular 5 to 90%, more particularly 10 to 80%, even more particularly 15 to 75%, more particularly still 20 to 70% by weight of (meth)acrylate-functionalized monomer based on the total weight of the curable composition. In particular, the curable composition of the invention may comprise 5 to 50% or 10 to 50% or 15 to 50% or 20 to 50% or 25 to 50% or 30 to 50%, by weight of (meth)acrylate-functionalized monomer based on the total weight of the curable composition. Alternatively, the curable composition of the invention may comprise 50 to 99.5% or 55 to 99.5% or 60 to 99.5% or 65 to 99.5% or 70 to 99.5%, by weight of (meth)acrylate-functionalized monomer based on the total weight of the curable composition.

In one embodiment, the ethylenically unsaturated compound comprises a (meth)acrylate-functionalized oligomer. The ethylenically unsaturated compound may comprise a mixture of (meth)acrylate-functionalized oligomers.

The (meth)acrylate-functionalized oligomer may be selected in order to enhance the flexibility, strength and/or modulus, among other attributes, of a cured polymer prepared using the curable composition of the present invention.

The (meth)acrylate functionalized oligomer may have 1 to 18 (meth)acrylate groups, in particular 2 to 6 (meth)acrylate groups, more particularly 2 to 6 acrylate groups.

The (meth)acrylate functionalized oligomer may have a number average molecular weight equal or more than 600 g/mol, in particular 800 to 15,000 g/mol, more particularly 1,000 to 5,000 g/mol.

In particular, the (meth)acrylate-functionalized oligomers may be selected from the group consisting of (meth)acrylate-functionalized urethane oligomers (sometimes also referred to as "urethane (meth)acrylate oligomers," "polyurethane (meth)acrylate oligomers" or "carbamate (meth)acrylate oligomers"), (meth)acrylate-functionalized epoxy oligomers (sometimes also referred to as "epoxy (meth)acrylate oligomers"), (meth)acrylate-functionalized polyether oligomers (sometimes also referred to as "polyether (meth)acrylate oligomers"), (meth)acrylate-functionalized polydiene oligomers (sometimes also referred to as "polydiene (meth)acrylate oligomers"), (meth)acrylate-functionalized polycarbonate oligomers (sometimes also referred to as "polycarbonate (meth)acrylate oligomers"), and (meth)acrylate-functionalized polyester oligomers (sometimes also referred to as "polyester (meth)acrylate oligomers") and mixtures thereof.

Preferably, the (meth)acrylate-functionalized oligomer comprises a (meth)acrylate-functionalized urethane oligomer, more preferably an acrylate-functionalized urethane oligomer.

Advantageously, the (meth)acrylate-functionalized oligomer comprises a (meth)acrylate-functionalized urethane oligomer having two (meth)acrylate groups, more preferably an acrylate-functionalized urethane oligomer having two acrylate groups.

Exemplary polyester (meth)acrylate oligomers include the reaction products of acrylic or methacrylic acid or mixtures or synthetic equivalents thereof with hydroxyl group-terminated polyester polyols. The reaction process may be conducted such that all or essentially all of the hydroxyl groups of the polyester polyol have been (meth)acrylated, particularly in cases where the polyester polyol is difunctional. The polyester polyols can be made by polycondensation reactions of polyhydroxyl functional components (in particular, diols) and polycarboxylic acid functional compounds (in particular, dicarboxylic acids and anhydrides). The polyhydroxyl functional and polycarboxylic acid functional components can each have linear, branched, cycloaliphatic or aromatic structures and can be used individually or as mixtures.

Examples of suitable epoxy (meth)acrylates include the reaction products of acrylic or methacrylic acid or mixtures thereof with an epoxy resin (polyglycidyl ether or ester). The epoxy resin may, in particular, by selected from bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolak resin, hydrogenated bisphenol A diglycidyl ether, hydrogenated bisphenol F diglycidyl ether, hydrogenated bisphenol S diglycidyl ether, 3,4-epoxycyclohexylmethyl-3',4'-epoxycyclohexanecarboxylate, 2-(3,4-epoxycyclohexyl-5,5-spiro-3,4-epoxy)cyclohexane-1,4-dioxane, bis(3,4-epoxycyclohexylmethyl)adipate, vinylcyclohexene oxide, 4-vinylepoxycyclohexane, bis(3,4-epoxy-6-methylcyclohexylmethyl)adipate,3,4-epoxy-6-methylcyclohexy 1-3',4'-epoxy-6'-methylcyclohexanecarboxylate, methylenebis(3,4-epoxycyclohexane), dicyclopentadiene diepoxide, di(3,4-epoxycyclohexylmethyl) ether of ethylene glycol, ethylenebis(3, 4-epoxycyclohexanecarboxylate), 1,4-butanediol diglycidyl ether, 1,6-hexanediol diglycidyl ether, glycerol triglycidyl ether, trimethylolpropane triglycidyl ether, polyethylene glycol diglycidyl ether, polypropylene glycol diglycidyl ether, polyglycidyl ethers of a polyether polyol obtained by the addition of one or more alkylene oxides to an aliphatic polyhydric alcohol such as ethylene glycol, propylene glycol, and glycerol, diglycidyl esters of aliphatic long-chain dibasic acids, monoglycidyl ethers of aliphatic higher alcohols, monoglycidyl ethers of phenol, cresol, butyl phenol, or polyether alcohols obtained by the addition of alkylene oxide to these compounds, glycidyl esters of higher fatty acids, epoxidized soybean oil, epoxybutylstearic acid, epoxyoctylstearic acid, epoxidized linseed oil, epoxidized polybutadiene, and the like.

Suitable polyether (meth)acrylate oligomers include, but are not limited to, the condensation reaction products of acrylic or methacrylic acid or synthetic equivalents or mixtures thereof with polyetherols which are polyether polyols (such as polyethylene glycol, polypropylene glycol or polytetramethylene glycol). Suitable polyetherols can be linear or branched substances containing ether bonds and terminal hydroxyl groups. Polyetherols can be prepared by ring opening polymerization of cyclic ethers such as tetrahydrofuran or alkylene oxides (e.g., ethylene oxide and/or propylene oxide) with a starter molecule. Suitable starter molecules include water, polyhydroxyl functional materials, polyester polyols and amines.

Polyurethane (meth)acrylate oligomers (sometimes also referred to as "urethane (meth)acrylate oligomers") suitable for use in the curable compositions of the present invention include urethanes based on aliphatic, cycloaliphatic and/or aromatic polyester polyols and polyether polyols and aliphatic, cycloalipahtic and/or aromatic polyester diisocyanates and polyether diisocyanates capped with (meth)acrylate end-groups. Suitable polyurethane (meth)acrylate oligomers include, for example, aliphatic polyester-based urethane di- and tetra-acrylate oligomers, aliphatic polyether-based urethane di- and tetra-acrylate oligomers, as well as aliphatic polyester/polyether-based urethane di- and tetra-acrylate oligomers.

The polyurethane (meth)acrylate oligomers may be prepared by reacting aliphatic, cycloaliphatic and/or aromatic polyisocyanates (e.g., diisocyanate, triisocyanate) with OH group terminated polyester polyols, polyether polyols, polycarbonate polyols, polycaprolactone polyols, polyorganosiloxane polyols (e.g., polydimethylsiloxane polyols), or polydiene polyols (e.g., polybutadiene polyols), or combinations thereof to form isocyanate-functionalized oligomers which are then reacted with hydroxyl-functionalized (meth)acrylates such as hydroxyethyl acrylate or hydroxyethyl methacrylate to provide terminal (meth)acrylate groups. For example, the polyurethane (meth)acrylate oligomers may contain two, three, four or more (meth)acrylate functional groups per molecule. Other orders of addition may also be practiced to prepare the polyurethane (meth)acrylate, as is known in the art. For example, the hydroxyl-functionalized (meth)acrylate may be first reacted with a polyisocyanate to obtain an isocyanate-functionalized (meth)acrylate, which may then be reacted with an OH group terminated polyester polyol, polyether polyol, polycarbonate polyol, polycaprolactone polyol, polydimethylsiloxane polyol, polybutadiene polyol, or a combination thereof. In yet another embodiment, a polyisocyanate may be first reacted with a polyol, including any of the aforementioned types of polyols, to obtain an isocyanate-functionalized polyol, which is thereafter reacted with a hydroxyl-functionalized (meth)acrylate to yield a polyurethane (meth)acrylate. Alternatively, all the components may be combined and reacted at the same time.

Suitable acrylic (meth)acrylate oligomers (sometimes also referred to in the art as "acrylic oligomers") include oligomers which may be described as substances having an oligomeric acrylic backbone which is functionalized with one or (meth)acrylate groups (which may be at a terminus of the oligomer or pendant to the acrylic backbone). The acrylic backbone may be a homopolymer, random copolymer or block copolymer comprised of repeating units of acrylic monomers. The acrylic monomers may be any monomeric (meth)acrylate such as Cl-C6 alkyl (meth)acrylates as well as functionalized (meth)acrylates such as (meth)acrylates bearing hydroxyl, carboxylic acid and/or epoxy groups. Acrylic (meth)acrylate oligomers may be prepared using any procedures known in the art, such as by oligomerizing monomers, at least a portion of which are functionalized with hydroxyl, carboxylic acid and/or epoxy groups (e.g., hydroxyalkyl(meth)acrylates, (meth)acrylic acid, glycidyl (meth)acrylate) to obtain a functionalized oligomer intermediate, which is then reacted with one or more (meth)acrylate-containing reactants to introduce the desired (meth)acrylate functional groups.

The curable composition of the invention may comprise 0 to 99.5%, in particular 5 to 90%, more particularly 10 to 80%, even more particularly 15 to 75%, more particularly still 20 to 70% by weight of (meth)acrylate-functionalized oligomer based on the total weight of the curable composition. In particular, the curable composition of the invention may comprise 5 to 50% or 10 to 50% or 15 to 50% or 20 to 50% or 25 to 50% or 30 to 50%, by weight of (meth)acrylate-functionalized oligomer based on the total weight of the curable composition. Alternatively, the curable composition of the invention may comprise 50 to 99.5% or 55 to 99.5% or 60 to 99.5% or 65 to 99.5% or 70 to 99.5%, by weight of (meth)acrylate-functionalized oligomer based on the total weight of the curable composition.

In one embodiment, the ethylenically unsaturated compound comprises an amine-modified acrylate. The ethylenically unsaturated compound may comprise a mixture of amine-modified acrylates.

An amine-modified acrylate is obtained by reacting an acrylate-functionalized compound with an amine-containing compound (aza-Michael addition). The amine-modified acrylate comprises at least one remaining acrylate group (i.e. an acrylate group that has not reacted with the amine-containing compound during the aza-Michael addition) and/or at least one (meth)acrylate group (which may not be reactive towards primary or secondary amines).

The acrylate-functionalized compound may be an acrylate-functionalized monomer and/or acrylate-functionalized oligomer as defined above.

The amine-containing compound comprises a primary or secondary amino group and optionally a tertiary amino group. The amine-containing compound may comprise more than one primary and/or secondary amino groups. The amine-containing compound may be selected from monoethanolamine (2-aminoethanol), 2-ethylhexylamine, octylamine, cyclohexylamine, sec-butylamine, isopropylamine, diethylamine, diethanolamine, dipropylamine, dibutylamine, 2-(methylamino)ethano-1,2-methoxyethylamine, bis(2-hydroxypropyl)amine, diisopropylamine, dipentylamine, dihexylamine, bis(2-ethylhexyl)amine, 1,2,3,4-tetrahydroisoquinoline, N-benzylmethylamine, morpholine, piperidine, dioctylamine, and di-cocoamine, dimethylaminopropylamine, dimethylaminopropylaminopropylamine, 1,4-bis(3-aminopropyl)piperazine, 1,3-bis(aminomethyl)cyclohexane, 1,4-bis(3-aminopropyl)piperazine, aniline and an optionally substituted benzocaine (ethyl-4-aminobenzoate).

Examples of commercially available amine-modified acrylates include CN3705, CN3715, CN3755, CN381 and CN386, all available from Arkema. Polymeric or multi-amino versions are also suitable.

The curable composition may comprise from 0% to 25%, in particular 2.5% to 20%, more particularly 5 to 15%, by weight of amine-modified acrylate based on the total weight of the curable composition.

In a first embodiment, the ethylenically unsaturated compound b) consists essentially of one or more (meth)acrylate-functionalized monomers as defined above and optionally one or more (meth)acrylate-functionalized oligomers as defined above.

In a second embodiment, the ethylenically unsaturated compound b) consists essentially of one or more (meth)acrylate-functionalized monomers as defined above and optionally one or more amine-modified acrylates as defined above.

In a third embodiment, the ethylenically unsaturated compound b) consists essentially of a mixture of one or more (meth)acrylate-functionalized monomers as defined above and one or more (meth)acrylate-functionalized oligomers as defined above.

In a fourth embodiment, the ethylenically unsaturated compound b) consists essentially of one or more (meth)acrylate-functionalized oligomers as defined above and optionally one or more (meth)acrylate-functionalized monomers as defined above.

In a fifth embodiment, the ethylenically unsaturated compound b) consists essentially of one or more (meth)acrylate-functionalized oligomers as defined above and optionally one or more amine-modified acrylates as defined above.

In a sixth embodiment, the ethylenically unsaturated compound b) consists essentially of a mixture of one or more (meth)acrylate-functionalized monomers as defined above and one or more (meth)acrylate-functionalized oligomers as defined above and one or more amine-modified acrylates as defined above.

### Amine synergist

The curable composition of the present invention may comprise an amine synergist. The curable composition may comprise a mixture of amine synergists.

Amine synergists may be introduced in the curable composition of the present invention in order to act synergistically with Norrish Type II photoinitiators and/or to reduce oxygen inhibition. Amine synergists are typically tertiary amines. When used in conjunction with Norrish Type II photoinitiators, the tertiary amine provides an active hydrogen donor site for the excited triple state of the photoinitiator, thus producing a reactive alkyl-amino radical which can subsequently initiate polymerization. Tertiary amines are also able to convert unreactive peroxy species, formed by reaction between oxygen and free radicals, to reactive alkyl-amino radicals, thus reducing the effects of oxygen on curing.

When the polymerizable composition comprises an amine-modified acrylate monomer or oligomer as defined above, an amine synergist may not need to be added to the composition.

When the composition comprises a cationically polymerizable compound, amine synergists may not be present.

Examples of suitable amine synergists include low-molecular weight tertiary amines (i.e. having a molecular weight of less than 200 g/mol) such as triethanol amine, N-methyldiethanol amine. Other types of amine synergists are aminobenzoates. Examples of aminobenzoates include ethyl 4-(dimethylamino)benzoate (EDB), pentyl 4-(dimethylamino)benzoate, 2-ethylhexyl 4-(dimethylamino)benzoate and 2-butoxyethyl 4-(dimethylamino)benzoate (BEDB).

The concentration of amine synergist in the curable composition will vary depending on the type of compound that is used. Typically, however, the curable composition is formulated to comprise from 0% to 25%, in particular 0.1% to 10%, more particularly 0.5 to 5%, by weight of amine synergist based on the total weight of the curable composition.

### Cationically polymerizable compound

The composition of the invention may further comprise a cationically polymerizable compound. The composition of the invention may comprise a mixture of cationically polymerizable compounds.

When the composition comprises a cationically polymerizable compound, the composition may be a hybrid free-radical/cationic composition, i.e. a composition that is cure by free radical polymerization and cationic polymerization.

The term "cationically polymerizable compound" means a compound comprising a polymerizing functional group which polymerizes via a cationic mechanism, for example a heterocyclic group or a carbon-carbon double bond substituted with an electrodonating group. In a cationic polymerization mechanism, a cationic initiator transfers charge to the cationically polymerizable compound which then becomes reactive and leads to chain growth by reaction with another cationically polymerizable compound.

The cationically polymerizable compound may be selected from epoxy-functionalized compounds, oxetanes, oxolanes, cyclic acetals, cyclic lactones, thiiranes, thiethanes, spiro orthoesters, ethylenically unsaturated compounds other than (meth)acrylates, derivatives thereof and mixtures thereof.

In a preferred embodiment, the cationically polymerizable compound may be selected from epoxy-functionalized compounds, oxetanes and mixtures thereof, in particular aromatic epoxy-functionalized compounds, cycloaliphatic epoxy-functionalized compounds, oxetanes and mixtures thereof.

Suitable epoxy-functionalized compounds capable of being cationically polymerized are glycidyl ethers, in particular mono-, di-, tri- and polyglycidyl ether compounds, and alicyclic ether compounds including those comprising residue of carboxylic acids such as, for example, alkylcarboxylic acid residual groups, alkylcycloalkylcarboxylic acid residual groups and dialkyl dicarboxylic acid residual groups. For example, the epoxy-functionalized compounds may be bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolak resin, hydrogenated bisphenol A diglycidyl ether, hydrogenated bisphenol F diglycidyl ether, hydrogenated bisphenol S diglycidyl ether, 3,4-epoxycyclohexylmethy1-3',4'-epoxycyclohexanecarboxylate, 2-(3,4-epoxycyclohexy1-5,5-spiro-3,4-epoxy)cyclohexane-1,4-dioxane, bis(3,4-epoxycyclohexylmethyl)adipate, vinylcyclohexene oxide, 4-vinylepoxycyclohexane, bis(3,4-epoxy-6-methylcyclohexylmethyl)adipate, 3,4-epoxy-6-methylcyclohexyl-3',4'-epoxy-6'-methylcyclohexanecarboxylate, methylenebis(3,4-epoxycyclohexane), dicyclopentadiene diepoxide, di(3,4-epoxycyclohexylmethyl) ether of ethylene glycol, ethylenebis(3, 4-epoxycyclohexanecarboxylate), epoxyhexahydrodioctylphthalate, epoxyhexahydro-di-2-ethylhexyl phthalate, 1,4-butanediol diglycidyl ether, 1,6-hexanediol diglycidyl ether, glycerol triglycidyl ether, trimethylolpropane triglycidyl ether, polyethylene glycol diglycidyl ether, polypropylene glycol diglycidyl ether, polyglycidyl ethers of polyether polyol obtained by the addition of one or more alkylene oxides to aliphatic polyhydric alcohols such as ethylene glycol, propylene glycol, and glycerol, diglycidyl esters of aliphatic long-chain dibasic acids, monoglycidyl ethers of aliphatic higher alcohols, monoglycidyl ethers of phenol, cresol, butyl phenol, or polyether alcohols obtained by the addition of alkylene oxide to these compounds, glycidyl esters of higher fatty acids, epoxidized soybean oil, epoxybutylstearic acid, epoxyoctylstearic acid, epoxidized linseed oil, epoxidized polybutadiene, and the like.

Suitable oxetanes capable of being cationically polymerized include trimethylene oxide, 3,3-dimethyloxetane, 3,3-dichloromethyloxetane, 3-ethyl-3-phenoxymethyloxetane, and bis(3-ethyl-3-methyloxy)butane, 3-ethyl-3-oxetanemethanol.

Suitable oxolanes capable of being cationically polymerized include tetrahydrofuran and 2,3-dimethyltetrahydrofuran.

Suitable cyclic acetals capable of being cationically polymerized include trioxane, 1,3-dioxolane, and 1,3,6-trioxanecyclooctane.

Suitable cyclic lactones capable of being cationically polymerized include β-propiolactone and ε-caprolactone.

Suitable thiiranes capable of being cationically polymerized include ethylene sulfide, 1,2-propylene sulfide, and thioepichlorohydrin.

Suitable thiethanes capable of being cationically polymerized include 3,3-dimethylthiethane.

Suitable spiro orthoesters capable of being cationically polymerized are compounds obtained by the reaction of an epoxy compound and a lactone.

Suitable ethylenically unsaturated compounds other than (meth)acrylates capable of being cationically polymerized include vinyl ethers such as ethylene glycol divinyl ether, triethylene glycol divinyl ether and trimethylolpropane trivinyl ether; aliphatic vinyl monomers such as vinylcyclohexane; olefins such isobutylene; dienes such as butadiene; vinyl alkyl ethers; vinyl aromatic monomers such as styrene and alkylstyrenes; unsaturated polymers such as polybutadiene; derivatives of the above organic substances; and the like, at least some of which may also be polymerizable by free radical mechanisms.

When the composition of the invention comprises a cationically polymerizable compound, it may further comprise a polyol. Suitable polyols are as defined above.

The curable composition of the invention may comprise 0 to 50%, in particular 5 to 40%, more particularly 10 to 30%, by weight of cationically polymerizable compound based on the total weight of the curable composition.

### Additives

The curable composition of the present invention may comprise an additive. The curable composition may comprise a mixture of additives.

In particular, the additive may be selected from sensitizers, stabilizers (antioxidants, light blockers/absorbers, polymerization inhibitors), foam inhibitors, flow or leveling agents, colorants, pigments, dispersants (wetting agents, surfactants), slip additives, fillers, chain transfer agents, thixotropic agents, matting agents, impact modifiers, waxes, mixtures thereof, and any other additive conventionally used in the coating, sealant, adhesive, molding, 3D printing or ink arts.

The curable composition may comprise a sensitizer.

Sensitizers may be introduced in the curable composition of the present invention in order to extend the sensitivity of the photoinitiator to longer wavelengths. For example, the sensitizer may absorb light at longer or shorter wavelengths than the photoinitiator and be capable of transferring the energy to the photoinitiator and revert to its ground state.

Examples of suitable sensitizers include anthracenes and carbazoles.

The concentration of sensitizer in the curable composition will vary depending on the photoinitiator that is used. Typically, however, the curable composition is formulated to comprise from 0% to 5%, in particular 0.1% to 3%, more particularly 0.5 to 2%, by weight of sensitizer based on the total weight of the curable composition.

The curable composition may comprise a stabilizer.

Stabilizers may be introduced in the curable composition of the present invention in order to provide adequate storage stability, processing stability, and shelf life. Advantageously, one or more such stabilizers are present at each stage of the method used to prepare the curable composition, to protect against unwanted reactions during processing of the ethylenically unsaturated components of the curable composition. As used herein, the term "stabilizer" means a compound or substance which retards or prevents reaction or curing of actinically-curable functional groups present in a composition in the absence of actinic radiation. However, it will be advantageous to select an amount and type of stabilizer such that the composition remains capable of being cured when exposed to actinic radiation (that is, the stabilizer does not prevent radiation curing of the composition). Typically, effective stabilizers for purposes of the present invention will be classified as free radical stabilizers (i.e., stabilizers which function by inhibiting free radical reactions).

Any of the stabilizers known in the art related to (meth)acrylate-functionalized compounds may be utilized in the present invention. Quinones represent a particularly preferred type of stabilizer which can be employed in the context of the present invention. As used herein, the term "quinone" includes both quinones and hydroquinones as well as ethers thereof such as monoalkyl, monoaryl, monoaralkyl and bis(hydroxyalkyl) ethers of hydroquinones. Hydroquinone monomethyl ether is an example of a suitable stabilizer which can be utilized. Other stabilizers known in the art such as hydroquinone (HQ), 4-tert-butylcatechol (TBC), 3,5-di-tertiobutyl-4-hydroxytoluene (BHT), phenothiazine (PTZ), pyrogallol, phosphite compounds, triphenyl antimony and tin(II) salts can also be used.

The concentration of stabilizer in the curable composition will vary depending upon the particular stabilizer or combination of stabilizers selected for use and also on the degree of stabilization desired and the susceptibility of components in the curable compositions towards degradation in the absence of stabilizer. Typically, however, the curable composition is formulated to comprise from 5 to 5000 ppm stabilizer. According to certain embodiments of the invention, the reaction mixture during each stage of the method employed to make the curable composition contains at least some stabilizer, e.g., at least 10 ppm stabilizer.

The polymerizable composition may comprise a colorant. A colorant may be a dye, a pigment and mixtures thereof. The term "dye", as used herein means a colorant having a solubility of 10 mg/L or more in the medium in which it is introduced at 25°C. The term "pigment" is defined in DIN 55943, as a colorant that is practically insoluble in the application medium under the pertaining ambient conditions, hence having a solubility of less than 10 mg/L therein at 25°C. The term "C.I." is used as an abbreviation for Colour Index.

The colorant may be a pigment. Organic and/or inorganic pigments may be used. If the colorant is not a self-dispersible pigment, the inkjet inks preferably also contain a dispersant, more preferably a polymeric dispersant. The pigment may be black, cyan, magenta, yellow, red, orange, violet, blue, green, brown and mixtures thereof. Pigments may be chosen from those disclosed by HERBST, Willy, et al. Industrial Organic Pigments, Production, Properties, Applications. 3rd edition. Wiley - VCH , 2004. ISBN 3527305769.

Particular pigments include:
- Carbon black;
- C.I. Pigment White 1, 3, 4, 5, 6, 7, 10, 11, 12, 14, 17, 18, 19, 21, 24, 25, 27, 28 and 32;
- C.I. Pigment Yellow 1 , 3, 10, 12, 13, 14, 17, 55, 65, 73, 74, 75, 83, 93, 97, 109, 111 , 120, 128, 138, 139, 150, 151 , 154, 155, 180,185 and 213;
- C.I. Pigment Red 17, 22, 23, 41 , 48:1 , 48:2, 49:1 , 49:2, 52:1 , 57:1 , 81 :1 , 81 :3, 88, 112, 122, 144, 146, 149, 169,170, 175, 176, 184, 185, 188, 202, 206, 207, 210, 216, 221 , 248, 251 , 254, 255, 264, 270 and 272;
- C.I. Pigment Violet 1 , 2, 19, 23, 32, 37 and 3;
- C.I. Pigment Blue 15:1 , 15:2, 15:3, 15:4, 15:6, 16, 56, 61 and (bridged) aluminium phthalocyanine pigments;
- C.I. Pigment Orange 5, 13, 16, 34, 40, 43, 59, 66, 67, 69, 71 and 73;
- C.I. Pigment Green 7 and 36;
- C.I. Pigment Brown 6 and 7;
and mixtures thereof.

The polymerizable composition of the invention may comprise a dispersant. The dispersant may be used to disperse an insoluble material such as a pigment or filler in the polymerizable composition.

The dispersant may be a polymeric dispersant, a surfactant and mixtures thereof.

Typical polymeric dispersants are copolymers of two, three, four, five or even more monomers. The properties of polymeric dispersants depend on both the nature of the monomers and their distribution in the polymer. Copolymeric dispersants preferably have the following polymer compositions:
- random copolymer (e.g. ABBAABAB);
- alternating copolymer (e.g. ABABABAB);
- gradient copolymer (e.g. AAABAABBABBB) ;
- block copolymers (e.g. AAAAABBBBBB);
- graft copolymers (polymeric backbone with polymeric side chains attached to the backbone); and mixed forms of these copolymers.

The polymeric dispersant may have a number average molecular weight Mn between 500 and 30000, more preferably between 1500 and 10000.

Commercial examples of polymeric dispersants include:
- DISPERBYK^{®} dispersants available from BYK CHEMIE GMBH;
- SOLSPERSE^{®} dispersants available from LUBRIZOL;
- TEGO^{®} DISPERSE dispersants from EVONIK;
- DISPEX^{®}, EFKA^{®} and JONCRYL^{®} dispersants from BASF;
- DISPONER^{®} dispersants from ELEMENTIS.

### Solvent

The curable composition of the invention may be solvent-based or water-based. As used herein, the term "solvent" means a non-reactive organic solvent, i.e. a solvent comprising carbon and hydrogen atom that does not react when exposed to the actinic radiation used to cure the curable compositions described herein.

Advantageously, the curable compositions of the present invention may be formulated to be solvent-free. For example, the curable compositions of the present invention may contain little or no solvent, e.g., less than 10% or less than 5% or less than 1% or even 0% by weight of solvent, based on the total weight of the curable composition.

### Formulations

The curable composition of the invention may be formulated as a one component or one part system. That is, the curable composition may be cured directly, i.e. it is not combined with another component or second part prior to being cured.

In a first embodiment, the curable composition of the invention may comprise:
a) a compound of formula (I) or a photoinitiator composition as defined above;
b) an ethylenically unsaturated compound;
c) optionally an amine synergist;
d) optionally a cationically polymerizable compound;
e) optionally an additive;
f) optionally a solvent.

The curable composition of the first embodiment may comprise or consist essentially of:
a) 0.05 to 30%, in particular 0.1 to 20%, more particularly 0.2 to 15%, of compound of formula (I) or photoinitiator composition as defined above;
b) 40 to 99.5%, in particular 50 to 95%, more particularly 60 to 90%, of ethylenically unsaturated compound;
c) 0 to 50%, in particular 5 to 40%, more particularly 10 to 30%, of cationically polymerizable compound;
d) 0 to 25%, in particular 0.1 to 10%, more particularly 0.5 to 5%, of amine synergist;
e) 0 to 30% of additive;
f) 0 to 30% of solvent;
wherein the % are % by weight based on the weight of the composition.

Preferably, the composition of the first embodiment does not comprise any component other than components a)-f). Accordingly, the total weight of components a), b), c), d), e) and f) may represent 100% of the weight of the composition.

In preferred embodiments of the invention, the curable composition is a liquid at 25°C. In various embodiments of the invention, the curable compositions described herein are formulated to have a viscosity of less than 10,000 mPa.s, or less than 5,000 mPa.s, or less than 1,000 mPa.s, or less than 500 mPa.s, or less than 250 mPa.s, or even less than 100 mPa.s as measured at 25°C using a Brookfield viscometer, model DV-II, using a 27 spindle (with the spindle speed varying typically between 20 and 200 rpm, depending on viscosity). In advantageous embodiments of the invention, the viscosity of the curable composition is from 10 to 10,000 mPa.s, or from 10 to 5,000 mPa.s, or from 10 to 1,000 mPa.s, or from 10 to 500 mPa.s, or from 10 to 250 mPa.s, or from 10 to 100 mPa.s at 25°C.

The curable compositions described herein may be compositions that are to be subjected to curing by means of free radical polymerization. In particular embodiments, the curable compositions may be photocured (i.e., cured by exposure to actinic radiation such as light, in particular visible or UV light). When the compound of formula (I) is a thioxanthone, the composition may be cured by a LED light source.

The curable composition of the invention may be an ink composition, an overprint varnish composition, a coating composition, an adhesive composition, a sealant composition, a molding composition, a dental composition, a cosmetic composition or a 3D-printing composition, in particular an ink composition

End use applications for the curable compositions include, but are not limited to, inks, coatings, adhesives, additive manufacturing resins (such as 3D printing resins), molding resins, sealants, composites, antistatic layers, electronic applications, recyclable materials, smart materials capable of detecting and responding to stimuli, packaging materials, personal care articles, cosmetics, articles for use in agriculture, water or food processing, or animal husbandry, and biomedical materials. The curable compositions of the invention thus find utility in the production of biocompatible articles. Such articles may, for example, exhibit high biocompatibility, low cytotoxicity and/or low extractables.

The composition according to the invention may in particular be used to obtain a cured product and a 3D printed article according to the following processes.

### Process for the preparation of a cured product and a 3D-printed article

The process for the preparation of a cured product according to the invention comprises curing the composition of the invention. In particular, the composition may be cured by exposing the composition to radiation. More particularly, the composition may be cured by exposing the composition to UV, near-UV and/or visible radiation. When the compound of formula (I) is a thioxanthone, the composition may be cured by exposing the composition to a LED light source, in particular a LED light source having an emission band in the range of 365-420 nm.

Curing may be accelerated or facilitated by supplying energy to the curable composition, such as by heating the curable composition. Thus, the cured product may be deemed as the reaction product of the curable composition, formed by curing. A curable composition may be partially cured by exposure to actinic radiation, with further curing being achieved by heating the partially cured article. For example, a product formed from the curable composition (e.g., a 3D printed article) may be heated at a temperature of from 40°C to 120°C for a period of time of from 5 minutes to 12 hours.

Prior to curing, the curable composition may be applied to a substrate surface in any known conventional manner, for example, by spraying, jetting, knife coating, roller coating, casting, drum coating, dipping, and the like and combinations thereof. Indirect application using a transfer process may also be used. A substrate may be any commercially relevant substrate, such as a high surface energy substrate or a low surface energy substrate, such as a metal substrate or plastic substrate, respectively. The substrates may comprise metal, paper, cardboard, glass, thermoplastics such as polyolefins, polycarbonate, acrylonitrile butadiene styrene (ABS), and blends thereof, composites, wood, leather and combinations thereof. When used as an adhesive, the curable composition may be placed between two substrates and then cured, the cured composition thereby bonding the substrates together to provide an adhered article. Curable compositions in accordance with the present invention may also be formed or cured in a bulk manner (e.g., the curable composition may be cast into a suitable mold and then cured).

The cured product obtained with the process of the invention may be an ink, a coating, a sealant, an adhesive, a molded article or a 3D-printed article. In particular, the cured product may be a 3D-printed article.

The 3D-printed article may, in particular, be obtained with a process for the preparation of a 3D-printed article that comprises printing a 3D article with the composition of the invention. In particular, the process may comprise printing a 3D article layer by layer or continuously.

A plurality of layers of a curable composition in accordance with the present invention may be applied to a substrate surface; the plurality of layers may be simultaneously cured (by exposure to a single dose of radiation, for example) or each layer may be successively cured before application of an additional layer of the curable composition.

The curable compositions which are described herein can be used as resins in three-dimensional printing applications. Three-dimensional (3D) printing (also referred to as additive manufacturing) is a process in which a 3D digital model is manufactured by the accretion of construction material. The 3D printed object is created by utilizing the computer-aided design (CAD) data of an object through sequential construction of two dimensional (2D) layers or slices that correspond to cross-sections of 3D objects. Stereolithography (SL) is one type of additive manufacturing where a liquid resin is hardened by selective exposure to a radiation to form each 2D layer. The radiation can be in the form of electromagnetic waves or an electron beam. The most commonly applied energy source is ultraviolet, visible or infrared radiation.

Sterolithography and other photocurable 3D printing methods typically apply low intensity light sources to radiate each layer of a photocurable resin to form the desired article. As a result, photocurable resin polymerization kinetics and the green strength of the printed article are important criteria if a particular photocurable resin will sufficiently polymerize (cure) when irradiated and have sufficient green strength to retain its integrity through the 3D printing process and post-processing.

The curable compositions of the invention are especially useful as 3D printing resin formulations, that is, compositions intended for use in manufacturing three-dimensional articles using 3D printing techniques. Such three-dimensional articles may be freestanding/self-supporting and may consist essentially of or consist of a composition in accordance with the present invention that has been cured. The three-dimensional article may also be a composite, comprising at least one component consisting essentially of or consisting of a cured composition as previously mentioned as well as at least one additional component comprised of one or more materials other than such a cured composition (for example, a metal component or a thermoplastic component or inorganic filler or fibrous reinforcement). The curable compositions of the present invention are particularly useful in digital light printing (DLP), although other types of three-dimensional (3D) printing methods may also be practiced using the inventive curable compositions (e.g., SLA, inkjet, multi-jet printing, piezoelectric printing, actinically-cured extrusion, and gel deposition printing). The curable compositions of the present invention may be used in a three-dimensional printing operation together with another material which functions as a scaffold or support for the article formed from the curable composition of the present invention.

Thus, the curable compositions of the present invention are useful in the practice of various types of three-dimensional fabrication or printing techniques, including methods in which construction of a three-dimensional object is performed in a step-wise or layer-by-layer manner. In such methods, layer formation may be performed by solidification (curing) of the curable composition under the action of exposure to radiation, such as visible, UV or other actinic irradiation. For example, new layers may be formed at the top surface of the growing object or at the bottom surface of the growing object. The curable compositions of the present invention may also be advantageously employed in methods for the production of three-dimensional objects by additive manufacturing wherein the method is carried out continuously. For example, the object may be produced from a liquid interface. Suitable methods of this type are sometimes referred to in the art as "continuous liquid interface (or interphase) product (or printing)" ("CLIP") methods. Such methods are described, for example, in WO 2014/126830; WO 2014/126834; WO 2014/126837; and Tumbleston et al., "Continuous Liquid Interface Production of 3D Objects," Science Vol. 347, Issue 6228, pp. 1349-1352 (March 20, 2015), the entire disclosure of which is incorporated herein by reference in its entirety for all purposes.

The curable composition may be supplied by ejecting it from a printhead rather than supplying it from a vat. This type of process is commonly referred to as inkjet or multijet 3D printing. One or more UV curing sources mounted just behind the inkjet printhead cures the curable composition immediately after it is applied to the build surface substrate or to previously applied layers. Two or more printheads can be used in the process which allows application of different compositions to different areas of each layer. For example, compositions of different colors or different physical properties can be simultaneously applied to create 3D printed parts of varying composition. In a common usage, support materials - which are later removed during post-processing - are deposited at the same time as the compositions used to create the desired 3D printed part. The printheads can operate at temperatures from about 25°C up to about 100°C. Viscosities of the curable compositions are less than 30 mPa.s at the operating temperature of the printhead.

The process for the preparation of a 3D-printed article may comprise the steps of:
a) providing (e.g., coating) a first layer of a curable composition in accordance with the present invention onto a surface;
b) curing the first layer, at least partially, to provide a cured first layer;
c) providing (e.g., coating) a second layer of the curable composition onto the cured first layer;
d) curing the second layer, at least partially, to provide a cured second layer adhered to the cured first layer; and
e) repeating steps c) and d) a desired number of times to build up the three-dimensional article.

After the 3D article has been printed, it may be subjected to one or more post-processing steps. The post-processing steps can be selected from one or more of the following steps removal of any printed support structures, washing with water and/or organic solvents to remove residual resins, and post-curing using thermal treatment and/or actinic radiation either simultaneously or sequentially. The post-processing steps may be used to transform the freshly printed article into a finished, functional article ready to be used in its intended application.

### Process of inkjet printing

The process of inkjet printing according to the invention comprised jetting the polymerizable composition of the invention onto a substrate.

The substrate on which the polymerizable composition is jetted may be any kind of substrate as defined above.

The polymerizable composition may be jetted by one or more print heads ejecting small droplets in a controlled manner through nozzles onto a substrate moving relative to the print head(s).

The print head may be a piezoelectric head or a continuous type print head.

The inkjet printing process may be carried out in a single pass or with a multi-pass printing mode.

The inkjet printing process may further comprise a UV-curing step. In inkjet printing, the UV curing device may be arranged in combination with the print head of the inkjet printer, travelling therewith so that the liquid UV curable inkjet ink is exposed to curing radiation very shortly after been jetted.

In a particularly preferred embodiment, the UV curing step is performed using UV LED light sources.

For facilitating curing, the inkjet printer may include one or more oxygen depletion units. The oxygen depletion units place a blanket of nitrogen or other relatively inert gas (e.g. CO₂), with adjustable position and adjustable inert gas concentration, in order to reduce the oxygen concentration in the curing environment.

### Uses

The compound of formula (I) as defined above or the photoinitiator composition as defined above may be used as a photoinitiating system in a radiation curable composition, in particular in a UV or LED-curable composition.

As used herein, "UV curable composition" means a composition that is at least partially cured by exposure to UV light emitted by a mercury light source, in particular a mercury-vapor lamp, and "LED-curable composition" means a composition that is at least partially cured by exposure to UV light emitted by a LED light source, in particular a LED light source having an emission band in the range of 365-420 nm.

Other curing methods may be combined with UV light, such as heating,

The compound of formula (I) as defined above or the photoinitiator composition as defined above may be used in a photopolymerization reaction. The photopolymerization reaction may be used to photopolymerize (e.g. cure) one or more ethylenically unsaturated compounds as defined above.

When the compound of formula (I) is liquid, it may advantageously be used to solubilize a photoinitiator other than a compound of formula (I).

The curable composition of the invention may be used to obtain an ink, a coating, a sealant, an adhesive, a molded article or a 3D-printed article, in particular an inkjet ink.

Within this specification, embodiments have been described in a way which enables a clear and concise specification to be written, but it is intended and will be appreciated that embodiments may be variously combined or separated without departing from the invention. For example, it will be appreciated that all preferred features described herein are applicable to all aspects of the invention described herein.

In some embodiments, the invention herein can be construed as excluding any element or process step that does not materially affect the basic and novel characteristics of the invention.

Additionally, in some embodiments, the invention can be construed as excluding any element or process step not specified herein.

Although the invention is illustrated and described herein with reference to specific embodiments, the invention is not intended to be limited to the details shown. Rather, various modifications may be made in the details within the scope and range of equivalents of the claims and without departing from the invention.

### Examples

### Materials

All materials used in the examples are readily available from standard commercial sources such as Sigma-Aldrich Company Ltd. and Tokyo Chemical Industry Ltd. unless otherwise specified. 2-(Chlorothio)benzoyl chloride was prepared according to the procedure given in US 4,101,558 (The Sherwin-Williams Company). Trimethylolpropane triacrylate, ethoxylated bisphenol A diacrylate and tripropylene glycol diacrylate are available from Sartomer.

### Synthesis of Photoinitiator 1: diethyl [(9-oxo-9H-thioxanthenyl)methyl]propanedioate as a mixture of isomers

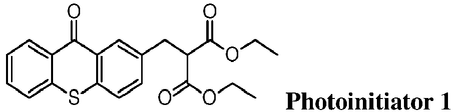

A solution of diethyl benzylmalonate (165 g, 0.659 mol) and 2-(chlorothio)benzoyl chloride (150 g, 0.725 mol) in dichloromethane (1 L) was added over 2 h to a stirred, ice-cooled suspension of anhydrous iron(III) chloride (246 g, 1.52 mol) in dichloromethane (1.2 L) and the resulting deep red solution was allowed to warm to room temperature and stirred for a further 16 h. The mixture was poured into ice water (∼2.5 L) and the biphasic mixture was mixed vigorously. The mixture was filtered to remove insoluble material and the layers were separated. The organic layer was collected and concentrated under reduced pressure (at 30-35 °C). To the crude residue was added *tert*-butyl methyl ether (1.2 L), and the resulting solution was washed with 5% hydrochloric acid (3 × 250 mL), water (200 mL), saturated aqueous sodium bicarbonate solution (3 × 300 mL) and brine (200 mL). The organic phase was dried (MgSO₄), filtered and concentrated under reduced pressure to give the desired **Photoinitiator 1** as a yellow/orange liquid (228 g, 90%, a mixture of isomers; the 2-isomer is the major product). ¹H NMR (400 MHz, CDCl₃); 8.63 (dd, *J* = 1.0 and 8.0 Hz, 1H), 8.49 (s, 1H), 7.65-7.40 (m, 5H), 4.24-4.12 (m, 4H), 3.75 (t, *J* = 8.0 Hz, 1H), 3.36 (d, *J* = 8.0 Hz, 2H), 1.27-1.18 (m, 6H). vₘₐₓ/cm⁻¹ 2981, 1727, 1636, 1591. EI-MS *m*/*z* 384.2 [M⁺].

### Synthesis of Intermediate 1: dibutyl benzylpropanedioate

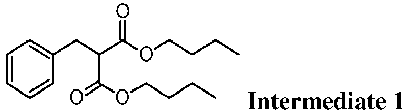

To a solution of benzylmalonic acid (2.0 g, 10.3 mmol) in 1-butanol (15 mL, 164 mmol) was added sulfuric acid (5 drops) and the mixture was heated at 60 °C for 5 h, then at 75 °C for 4.5 h. The mixture was allowed to cool to room temperature, then was poured into ethyl acetate (50 mL) and saturated aqueous sodium bicarbonate solution (50 mL) and the layers were separated. The organic phase was washed with saturated aqueous sodium bicarbonate solution (50 mL), water (50 mL) and brine (30 mL), then was dried (MgSO₄), filtered and concentrated under reduced pressure to give the desired **Intermediate 1** as a colourless liquid (2.6 g, 83%). ¹H NMR (400 MHz, CDCl₃); 7.29-7.18 (m, 5H), 4.11-4.06 (m, 4H), 3.66 (t, *J* = 8.0 Hz, 1H), 3.21 (t, *J* = 8.0 Hz, 2H), 1.59-1.52 (m, 4H), 1.34-1.25 (m, 4H), 0.89 (t, *J* = 7.5 Hz, 6H). *v*ₘₐₓ/cm⁻¹ 2959, 2874, 1731. EI-MS *m*/*z* 306.2 [M⁺].

### Synthesis of Photoinitiator 2: dibutyl [(9-oxo-9H-thioxanthenyl)methyl]propanedioate as a mixture of isomers

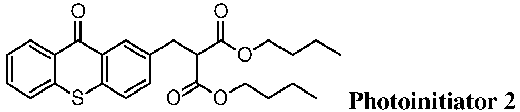

A solution of **Intermediate 1** (1.80 g, 5.88 mmol) and 2-(chlorothio)benzoyl chloride (1.40 g, 6.76 mmol) in dichloromethane (12 mL) was added over 10 minutes to an ice-cooled suspension of iron(III) chloride (2.86 g, 17.6 mmol) in dichloromethane (13 mL) and the resulting deep red solution was allowed to warm to room temperature and stirred for 16 h. The mixture was poured into ice water (30 mL) and the biphasic mixture was mixed vigorously. The mixture was filtered to remove insoluble material and the layers were separated. The organic layer was collected and concentrated under reduced pressure (at 30-35 °C). To the crude residue was added *tert*-butyl methyl ether (50 mL),and the resulting solution was washed with 5% hydrochloric acid (3 × 25 mL), water (20 mL), saturated aqueous sodium bicarbonate solution (3 × 30 mL) and brine (20 mL). The organic phase was dried (MgSO₄), filtered and concentrated under reduced pressure to give the desired **Photoinitiator 2** as a yellow/orange liquid (2.1 g, 81%, a mixture of isomers; the 2-isomer is the major product). ¹H NMR (400 MHz, CDCl₃); 8.60 (dd, *J* = 1.5 and 8.0 Hz, 1H), 8.46 (s, 1H), 7.63-7.45 (m, 5H), 4.14-4.09 (m, 4H), 3.74 (t, *J* = 8.0 Hz, 1H), 3.34 (d, *J* = 8.0 Hz, 2H), 1.60-1.53 (m, 4H), 1.35-1.24 (m, 4H), 0.91-0.85 (m, 6H). *v*ₘₐₓ/cm⁻¹ 2958, 2873, 1728, 1637, 1592. EI-MS *m*/*z* 440.2 [M⁺].

### Synthesis of Photoinitiator 3 as a mixture of isomers

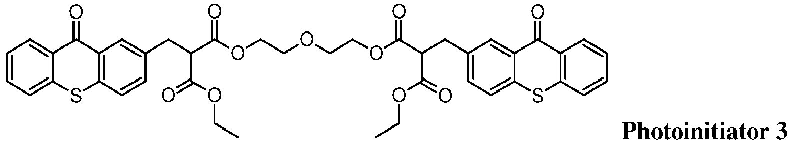

To a solution of **Photoinitiator 1** (5.0 g, 13.0 mmol) and diethylene glycol (0.69 g, 6.50 mmol) in toluene (60 mL) were added 4Å molecular sieves (6.0 g) and titanium(IV) isopropoxide (300 mg, 1.06 mmol) and the resulting mixture was heated at 110 °C for 8 h. The mixture was allowed to cool to room temperature, then water (50 mL) and toluene (50 mL) were added. The layers were separated, and the organic layer was washed with saturated aqueous sodium bicarbonate solution (50 mL) and brine (50 mL). The organic phase was dried (MgSO₄), filtered and concentrated under reduced pressure to give the crude product. Column chromatography (eluting with 1:1 petrol-ethyl acetate) gave the desired **Photoinitiator 3** as a yellow liquid (1.04 g, 20%, a mixture of isomers). ¹H NMR (400 MHz, CDCl₃); 8.60-8.55 (m, 2H), 8.45 (s, 2H), 7.63-7.45 (m, 10H), 4.26-4.10 (m, 8H), 3.79-3.70 (m, 2H), 3.63-3.38 (m, 4H), 3.36-3.32 (m, 4H), 1.29-1.14 (m, 6H). *v*ₘₐₓ/cm⁻¹ 2979, 1727, 1635, 1591. ESI-MS *m*/*z* 783.2 [MH⁺], 805.2 [MNa⁺].

### Synthesis of Photoinitiator 4: diethyl [(benzoylphenyl)methyl]propanedioate as a mixture of isomers

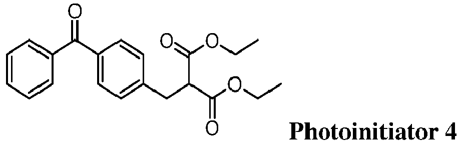

A solution of diethyl benzylmalonate (5.0 g, 20.0 mmol) and benzoyl chloride (3.4 g, 24.0 mmol) in dichloromethane (25 mL) was added over 5 mins to a stirred, ice-cooled suspension of anhydrous iron(III) chloride (9.7 g, 59.9 mmol) in dichloromethane (40 mL) and the resulting red/brown solution was allowed to warm to room temperature and stirred for a further 16 h. Extra benzoyl chloride (0.3 g) was added and the mixture was stirred at 40 °C for a further 4 h. The mixture was allowed to cool to room temperature and water (100 mL) was added. The resulting biphasic mixture was concentrated under reduced pressure at 35 °C to remove dichloromethane, and toluene (100 mL) was added to the aqueous slurry with thorough mixing. The layers were separated and the organic phase was washed with 5% hydrochloric acid (100 mL), saturated aqueous sodium bicarbonate solution (2 × 60 mL), and brine (50 mL). The organic phase was dried (MgSO₄), filtered and concentrated under reduced pressure to give the crude product. Column chromatography (eluting with 3:1 petrol-ethyl acetate) gave the pure **Photoinitiator 4** as a pale orange liquid (3.0 g, 42%, a mixture of isomers; the 4-isomer is the major product). ¹H NMR (400 MHz, CDCl₃); 7.78-7.72 (m, 4H), 7.60-7.56 (m, 1H), 7.47 (t, *J* = 7.5 Hz, 2H), 7.32 (d, *J* = 8.0 Hz, 2H), 4.22-4.12 (m, 4H), 3.69 (t, *J* = 8.0 Hz, 1H), 3.30 (d, *J* = 8.0 Hz, 2H), 1.22 (t, *J* = 7.0 Hz, 6H). *v*ₘₐₓ/cm⁻¹ 2982, 1728, 1656, 1607. EI-MS *m*/*z* 354.2 [M⁺].

### Curing Experiments

The following example illustrates the high curing speed of the photoinitiators disclosed in this invention in comparison with existing commercially available thioxanthone photoinitiators SpeedCure^{®} 2-ITX (Arkema) and Omnipol^{®} TX (IGM resins) and benzophenone photoinitiators Speedcure^{®} BP (Arkema) and Speedcure^{®} 7005 (Arkema).

Formulations were prepared containing 2% w/w of each photoinitiator and 5% w/w SpeedCure^{®} EDB (ethyl 4-(N,N'-dimethylamino)benzoate from Arkema, as co-initiator) in a 7:3 w/w mixture of ethoxylated bisphenol A diacrylate and tripropylene glycol diacrylate, with stirring at 30-40 °C until all samples were fully homogeneous; the formulations were then allowed to cool to room temperature. The photoinitiators and co-initiators remained fully soluble in the resin formulations throughout these experiments.

Formulations were then cured on Leneta Form 3N-31 gloss finish paper at 6 and 24 µm film thicknesses using a belt-cure instrument; the films were prepared using appropriate K-bars. Thioxanthone **Photoinitiator 1** and **Photoinitiator 2** were cured in comparison to Speedcure^{®} 2-ITX and Omnipol^{®} TX under a Hg lamp (20 m/min belt speed) and LED lamps at 365 and 395 nm (15 m/min belt speed). Benzophenone **Photoinitiator 4** was cured in comparison to Speedcure^{®} BP and Speedcure^{®} 7005 under a Hg lamp (20 m/min belt speed); LED curing was not attempted for any of the benzophenone photoinitiators. The cure speed for each formulation was calculated from the number of passes under each lamp required to give complete surface cure (determined when light scratching of the surface of the film no longer left a mark) and depth cure (determined using the 'thumb-twist' test, where no visible mark is made when a thumb is pressed down firmly onto the coating with a twisting motion) for each formulation, and from the belt speed; the calculated cure speed (in m/min) is given in Tables 1-4. Where cure speed was less than 0.6 m/min for Hg lamp curing or less than 0.3 m/min for LED lamp curing, the result was recorded as 'No cure'.

**Table 1 - Curing of Thioxanthone Photoinitiators under Hg Lamp, 20 m/min belt speed**

| **Photoinitiator*** | **Cure Speed (m/min)** | | | |
|---|---|---|---|---|
| | 6 µm film thickness | | 24 µm film thickness | |
| | Surface | Depth | Surface | Depth |
| SpeedCure^{®} 2-ITX | 6.7 | 5.0 | 6.7 | 5.0 |
| **Photoinitiator 1** | 5.0 | 4.0 | 5.0 | 4.0 |
| **Photoinitiator 2** | 4.3 | 3.2 | 5.0 | 3.3 |
| Omnipol^{®} TX | 4.0 | 3.3 | 4.7 | 3.8 |

| | | | | |
|---|---|---|---|---|
| *Photoinitiator present at 2% w/w concentration in 7:3 ethoxylated bisphenol A diacrylate:tripropylene glycol diacrylate. Formulations also contained 5% w/w SpeedCure^{®} EDB as amine co-initiator. | | | | |

**Table 2 - Curing of Thioxanthone Photoinitiators under LED Lamp at 365 nm, 15 m/min belt speed**

| **Photoinitiator*** | **Cure Speed (m/min)** | |
|---|---|---|
| | 24 µm film thickness | |
| | Surface | Depth |
| SpeedCure^{®} 2-ITX | 5.8 | 1.9 |
| **Photoinitiator 1** | 5.0 | 1.1 |
| **Photoinitiator 2** | 0.9 | No cure |
| Omnipol^{®} TX | 2.0 | 0.5 |

| | | |
|---|---|---|
| *Photoinitiator present at 2% w/w concentration in 7:3 ethoxylated bisphenol A diacrylate:tripropylene glycol diacrylate. Formulations also contained 5% w/w SpeedCure^{®} EDB as amine co-initiator. | | |

**Table 3 - Curing of Thioxanthone Photoinitiators under LED Lamp at 395 nm, 15 m/min belt speed**

| **Photoinitiator*** | **Cure Speed (m/min)** | |
|---|---|---|
| | 24 µm film thickness | |
| | Surface | Depth |
| SpeedCure^{®} 2-ITX | 5.0 | 2.1 |
| **Photoinitiator 1** | 4.6 | 1.3 |
| **Photoinitiator 2** | 0.9 | No cure |
| Omnipol^{®} TX | 3.8 | 0.7 |

| | | |
|---|---|---|
| *Photoinitiator present at 2% w/w concentration in 7:3 ethoxylated bisphenol A diacrylate:tripropylene glycol diacrylate. Formulations also contained 5% w/w SpeedCure^{®} EDB as amine co-initiator. | | |

**Table 4 - Curing of Benzophenone Photoinitiators under Hg, 20 m/min belt speed**

| **Photoinitiator*** | **Cure Speed (m/min)** | | | |
|---|---|---|---|---|
| | 6 µm film thickness | | 24 µm film thickness | |
| | Surface | Depth | Surface | Depth |
| SpeedCure^{®} BP | 2.5 | 1.9 | 3.3 | 3.3 |
| **Photoinitiator 4** | 1.2 | 1.0 | 2.2 | 2.0 |
| SpeedCure^{®} 7005 | No cure | No cure | No cure | No cure |

| | | | | |
|---|---|---|---|---|
| *Photoinitiator present at 2% w/w concentration in 7:3 ethoxylated bisphenol A diacrylate:tripropylene glycol diacrylate. Formulations also contained 5% w/w SpeedCure^{®} EDB as amine co-initiator. | | | | |

As can be seen from these results, the liquid Type-II photoinitiators disclosed in this invention are effective photoinitiators for the rapid UV-curing of common acrylate resin systems when used in the presence of a suitable amine co-initiator. The compounds disclosed in this invention give comparably rapid curing to existing commercially available photoinitiators in some cases.

### Solubility Experiments

The following example demonstrates the high solubility of a liquid photoinitiator disclosed in this invention in comparison with existing commercially available solid photoinitiators Speedcure^{®} 2-ITX (Arkema) and Speedcure^{®} DETX (Arkema).

Each photoinitiator - liquid thioxanthone **Photoinitiator 1,** Speedcure^{®} 2-ITX, or Speedcure^{®} DETX, 1.0 g in all cases - was dissolved in an acrylate resin - trimethylolpropane triacrylate (TMPTA), tripropylene glycol diacrylate (TPGDA), or 1,6-hexanediol diacrylate (HDDA), 9.0 g in each case - with stirring at 25 °C for 30 mins, giving 10% w/w solutions. In all cases the photoinitiators remained fully dissolved for 24 h. Extra portions of photoinitiator were then added with stirring at 25 °C so that the concentration of the photoinitiator in each resin increased by 2% w/w with each addition; after the addition of each portion, stirring at 25°C was continued for 24 h. The maximum solubility was judged to have been reached when the extra 2% portion either would not dissolve within 24 h, or when the photoinitiator either precipitated or settled out of solution. These maximum solubility limits are given in Table 5. Where a range is given, the photoinitiator was fully soluble at the lower value, and remained in solution for 24 h, but was seen to crystallize out of solution at the higher value within 24 h. Solubility above 30% w/w was not tested.

**Table 5 - Solubility of Photoinitiators in Common Acrylate Resins**

| **Photoinitiator** | **Maximum Solubility (% w/w at 25 °C)*** | | |
|---|---|---|---|
| | TMPTA | TPGDA | HDDA |
| **Photoinitiator 1** | > 30 | > 30 | > 30 |
| SpeedCure^{®} 2-ITX | 18-20 | 20-22 | 28-30 |
| SpeedCure^{®} DETX | 10-12 | 12-14 | 18-20 |

| | | | |
|---|---|---|---|
| *Solubility above 30% w/w was not tested. TMPTA = trimethylolpropane triacrylate, TPGDA = tripropylene glycol diacrylate, HDDA = 1,6-hexanediol diacrylate | | | |

As can be seen from these results, the novel liquid thioxanthone **Photoinitiator 1** has extremely high solubility in a range of acrylate resins; it remained fully dissolved for > 1 week. The solubility of the liquid thioxanthone disclosed by this invention is higher than that of existing commercial solid thioxanthones, which were observed to crystallise out of solution at higher concentrations.

## Claims

1. A compound of general formula (I) 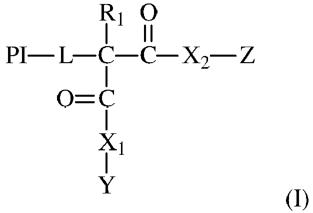 wherein
each PI is independently a photoinitiator moiety comprising an arylketone moiety;
each L is independently a linker selected from a direct bond, -C(=O)- or a divalent linker comprising 1 to 6, in particular 1 to 4, more particularly 1 or 2, carbon atoms;
each R₁ is independently H, alkyl or aryl;
each X₁ and X₂ is independently O, NR₂, N(R₂)₃ or S;
each R₂ is independently H or an optionally substituted alkyl;
each Y and Z are independently selected from H, an ionic moiety and an organic group, or Y and Z may form, together with the atoms to which they are attached, a ring.

2. The compound of claim 1, wherein each PI is independently a photoinitiator moiety comprising an arylketone moiety selected from a thioxanthone moiety, a benzophenone moiety, an anthraquinone moiety, a xanthone moiety, an anthrone moiety and an acridone moiety;
in particular each PI is independently a photoinitiator moiety of formula (II) or (III): 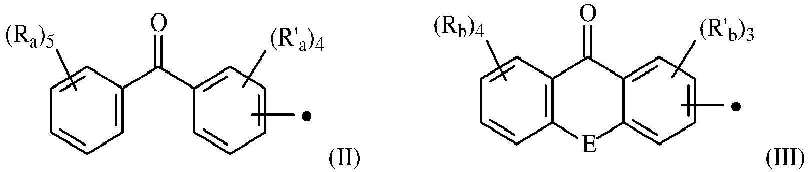 wherein
E is S, O, C(=O), CR₀R'₀ or NR₃, in particular S;
R₀, R'₀ and R₃ are independently H, an optionally substituted alkyl or an optionally substituted aryl;
the symbol • represents a point of attachment to linker L of formula (I);
Rₐ, R'ₐ, R_{b} and R'_{b} are independently selected from H, halogen, alkyl, cycloalkyl, heterocycloalkyl, alkoxy, aryloxy, thioalkyl, thioaryl, alkenyl, alkynyl, aryl, aralkyl, alkaryl, heteroaryl, -C(=O)R₄, -NR₅R₆, alkylamino, alkylthiol, hydroxyalkyl, haloalkyl, -NO₂, -CN, - C(=O)OR₇, -C(=O)NR₅R₆, -OH, -SH, and a group of formula (IV): 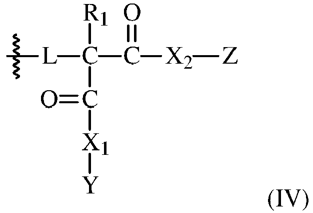
wherein L, R₁, X₁, X₂ ,Y and Z are as defined in claim 1;
two adjacent Rₐ, R'ₐ, R_{b} and R'_{b} groups may form, with the carbon atoms to which they are attached, a 5 to 8 membered ring;
R₄ is selected from an optionally substituted alkyl, an optionally substituted cycloalkyl, an optionally substituted heterocycloalkyl and an optionally substituted aryl;
R₅, R₆ and R₇ are independently selected from H, alkyl and aryl.

3. The compound of claim 2, wherein each PI is independently a benzophenone moiety of formula (IIa), (IIb), (IIc) or (IId) or a thioxanthone moiety of formula (IIIa), (IIIb), (IIIc) or (IIId): 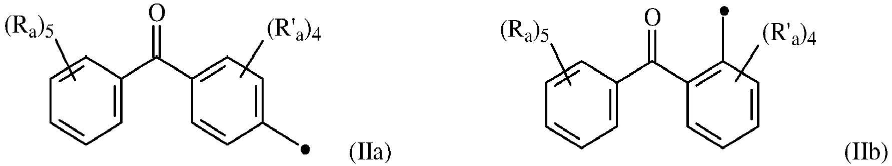 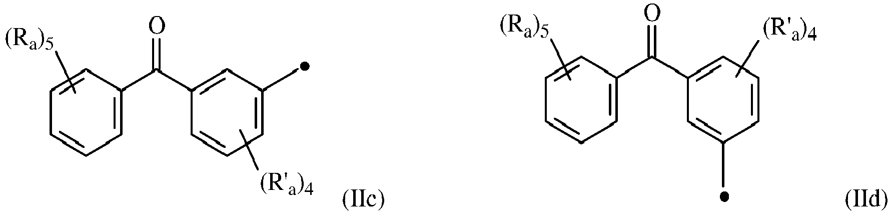 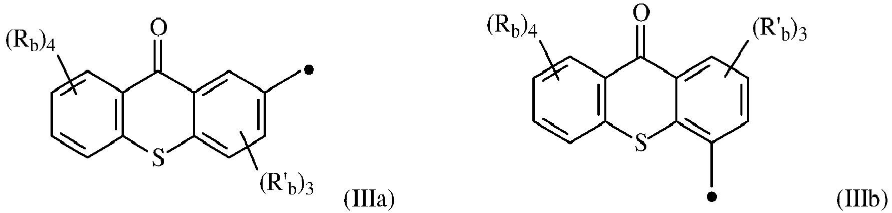 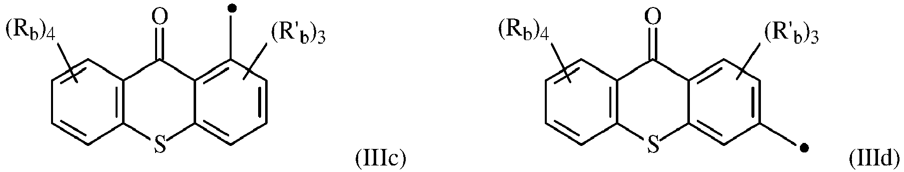
wherein Rₐ, R'ₐ, R_{b}, R'_{b} and symbol • are as defined in claim 2;
in particular each PI is independently a benzophenone moiety of formula (IIa) or (IIb) or a thioxanthone moiety of formula (IIIa) or (IIIb);
more particularly each PI is independently a benzophenone moiety of formula (IIa) or (IIb) or a thioxanthone moiety of formula (IIIa) or (IIIb) and Rₐ, R'ₐ, R_{b} and R'_{b} are all H.

4. The compound according to any one of claims 1 to 3, wherein each L is a linker independently selected from direct bond, -(C=O)-, alkylene, oxyalkylene, thioalkylene, ketoalkylene, alkenylene, arylene;
in particular each L is a linker independently selected from a direct bond, -(C=O)-, an alkylene of formula (V), an oxyalkylene of formula (VI), a thioalkylene of formula (VII) and a ketoalkylene of formula (VIII) 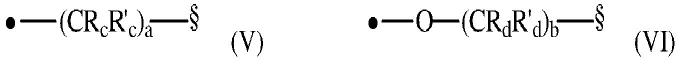 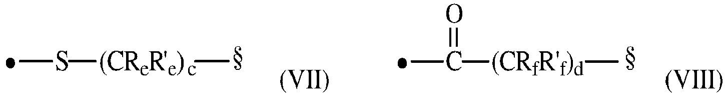 wherein
each R_{c}, R'_{c}, R_{d}, R'_{d}, Rₑ, R'ₑ, R_{f} and R'_{f} is independently H or alkyl, in particular H;
a, b and c are independently 1, 2, 3, 4, 5 or 6, in particular 1 or 2, more particularly 1;
d is 1, 2, 3, 4 or 5, in particular 1 or 2, more particularly 1
the symbol • represents a point of attachment to the PI moiety of formula (I);
the symbol § represents a point of attachment to the C(R₁) moiety of formula (I);
more particularly each L is independently an alkylene of formula (V) wherein
R_{c} and R'_{c} are independently H or alkyl, in particular H;
a is 1, 2, 3, 4, 5 or 6, in particular 1 or 2, more particularly 1.

5. The compound according to any one of claims 1 to 4, wherein R₁ is H.

6. The compound according to any one of claims 1 to 5, wherein X₁ and X₂ are both O.

7. The compound according to any one of claims 1 to 6, wherein each Y and Z is independently selected from H, an ionic moiety, an optionally substituted alkyl, an alkoxylated alkyl, a polymeric backbone, a polymeric core and a group of formula (IX): 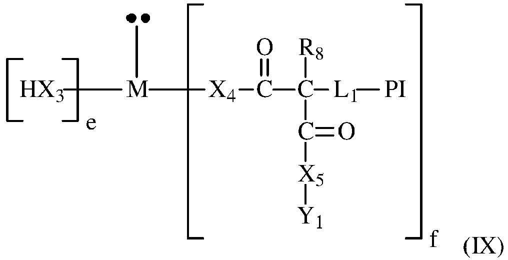 wherein
M is a (e+f)valent linker;
each PI is independently a photoinitiator moiety comprising an arylketone;
each L₁ is independently a linker selected from a direct bond, -C(=O)- or a divalent linker comprising 1 to 6, in particular 1 to 4, more particularly 1 or 2, carbon atoms;
each R₈ is independently H, alkyl or aryl;
each X₃, X₄ and X₅ is independently O, NR₉ or S;
each R₉ is independently H or an optionally substituted alkyl;
each Y₁ is independently H, an optionally substituted alkyl, an alkoxylated alkyl or Y₁ may form a ring with Y, Z or another Y₁;
e is from 0 to 6;
f is from 0 to 6;
with the proviso that at least one of e and f is not 0;
symbol •• represents a point of attachment to X₁ or X₂.

8. The compound according to any one of claims 1 to 7, wherein the compound is liquid at 20°C or soluble at 25°C in a substance selected from a non-reactive solvent, a (meth)acrylate monomer, a (meth)acrylate oligomer and mixtures thereof.

9. A photoinitiator composition comprising a mixture of at least two distinct compounds of formula (I) according to any one of claims 1 to 8.

10. A photoinitiator composition comprising a compound of formula (I) according to any one of claims 1 to 8 and a photoinitiator other than a compound of formula (I).

11. A process for photopolymerizing one or more ethylenically unsaturated compounds comprising contacting one or more ethylenically unsaturated compounds with a compound of formula (I) according to any one of claims 1 to 8 or a photoinitiator composition according to claim 9 or 10, and irradiating the mixture, in particular with visible, near-UV and/or UV light, more particularly with a LED light source.

12. A curable composition comprising:
a) a compound of formula (I) according to any one of claims 1 to 8 or a photoinitiator composition according to claim 9 or 10; and
b) an ethylenically unsaturated compound.

13. The curable composition of claim 12, wherein the ethylenically unsaturated compound is selected from a (meth)acrylate functionalized monomer, a (meth)acrylate functionalized oligomer, an amine-modified acrylate and mixtures thereof.

14. The polymerizable composition of claim 12 or 13, wherein the curable composition comprises:
- from 0.05 to 30%, from 0.1 to 20%, from 0.2 to 15%, from 0.5 to 10% or from 1 to 5% of component a);
- from 70 to 99,95%, from 80 to 99.9%, from 85 to 99.8%, from 90 to 99.5% or from 95 to 99% of component b);
the % being % by weight based on the total weight of components a) and b).

15. The curable composition of any one of claims 12 to 14, wherein the curable composition is an ink composition, a coating composition, an adhesive composition, a sealant composition, a molding composition, a dental composition, a cosmetic composition or a 3D-printing composition, in particular an ink composition.

16. A process for the preparation of a cured product, comprising curing the curable composition according to any one of claims 12 to 15, in particular by exposing the curable composition to radiation such as UV, near-UV and/or visible radiation, more particularly by exposing the curable composition to a LED light source.

17. A process of inkjet printing comprising jetting the curable composition according to any one of claims 12 to 15 onto a substrate.

18. A use of a compound of formula (I) according to any one of claims 1 to 8 or a photoinitiator composition according to claim 9 or 10, as a photoinitiator or a photoinitiating system in a radiation curable composition, in particular in a UV or LED-curable composition.

19. A use of a compound of formula (I) according to any one of claims 1 to 8 or a photoinitiator composition according to claim 9 or 10, in a photopolymerization reaction.

20. A use of a compound of formula (I) according to any one of claims 1 to 8 or a photoinitiator composition according to claim 9, to solubilize a photoinitiator other than a compound of formula (I).
